# EUROPEAN PATENT APPLICATION

(11) **EP 0 974 600 A2**
(43) Date of publication of application: **26.01.2000**
(21) Application number: 99304977.4
(22) Date of filing: 24.06.1999
(51) Int. Cl.: C07K 16/24, C12N 15/13, A61K 39/395

(54) **Anti-interleukin-18 antibody**

(30) Priority: 24.06.1998 JP 17758098; 12.10.1998 JP 28904498; 22.12.1998 JP 36502398
(71) Applicant: KABUSHIKI KAISHA HAYASHIBARA SEIBUTSU KAGAKU KENKYUJO, Okayama-shi Okayama (JP)
(72) Inventor: Nishida, Yoshihiro, Okayama (JP); Okura, Takanori, Kurashiki-shi, Okayama (JP); Tanimoto, Tadao, Okayama-shi, Okayama (JP); Kurimoto, Masashi, Okayama-shi, Okayama (JP)
(74) Representative: Daniels, Jeffrey Nicholas

(57) **Abstract**

Disclosed are artificially produced peptide capable of neutralizing the biological activities of IL-18, which comprises a part or the whole of the variable regions in anti-interleukin-18 antibody, including single chain variable region fragments and humanized antibodies, a process of producing the peptide, and uses thereof. The peptide is useful as pharmaceutical to treat and prevent diseases such as autoimmune diseases and inflammatory diseases, where the biological activities of interleukin-18 are involved.

### Annex to the description

SEQUENCE LISTING

<110> Kabushiki Kaisha Hayashibara Seibutsu Kagaku Kenkyujo

<120> PEPTIDE

<130>

<150> JP 177,580/98
<151> 1998-6-24

<150> JP 289,044/98
<151> 1998-10-12

<150> JP 365,023/98
<151> 1998-12-22

<160> 33

<210> 1
<211> 108
<212> PRT
<213> Mus musculus

<400> 1
Asp Ile Gln Met Thr Gln Ser Pro Ser Ser Leu Ser Ala Ser Leu Gly
 1               5                  10                  15

Glu Arg Val Ser Leu Thr Cys Arg Ala Ser Gln Asp Ile Gly Ser Lys
            20                  25                  30

Leu Tyr Trp Leu Gln Gln Glu Pro Asp Gly Thr Phe Lys Arg Leu Ile
        35                  40                  45

Tyr Ala Thr Ser Ser Leu Asp Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Val Asp Tyr Tyr Cys Leu Gln Tyr Ala Ser Ser Pro Tyr
                85                  90                  95

Thr Phe Gly Gly Gly Thr Lys Leu Ala Ile Lys Arg
            100                 105

<210> 2
<211> 113
<212> PRT
<213> Mus musculus

<400> 2
Glu Ile Gln Leu Gln Gln Ser Gly Pro Glu Leu Val Lys Pro Gly Ala
 1               5                  10                  15

Ser Val Lys Val Ser Cys Lys Ala Ser Gly Tyr Ser Phe Thr Asp Tyr

20                  25                  30

Phe Ile Tyr Trp Val Lys Gln Ser His Gly Lys Ser Leu Glu Trp Ile
        35                  40                  45

Gly Asp Ile Asp Pro Tyr Asn Gly Asp Thr Ser Tyr Asn Gln Lys Phe
    50                  55                  60

Arg Asp Lys Ala Thr Leu Thr Val Asp Gln Ser Ser Thr Thr Ala Phe
65                  70                  75                  80

Met His Leu Asn Ser Leu Thr Ser Glu Asp Ser Ala Val Tyr Phe Cys
                85                  90                  95

Ala Arg Gly Leu Arg Phe Trp Gly Gln Gly Thr Leu Val Thr Val Ser
            100                 105                 110

Ala


<210> 3
<211> 11
<212> PRT
<213> Mus musculus

<400> 3
Arg Ala Ser Gln Asp Ile Gly Ser Lys Leu Tyr
  1               5                  10

<210> 4
<211> 7
<212> PRT
<213> Mus musculus

<400> 4
Ala Thr Ser Ser Leu Asp Ser
  1               5

<210> 5
<211> 9
<212> PRT
<213> Mus musculus

<400> 5
Leu Gln Tyr Ala Ser Ser Pro Tyr Thr
  1               5

<210> 6
<211> 10
<212> PRT
<213> Mus musculus

<400> 6
Gly Tyr Ser Phe Thr Asp Tyr Phe Ile Tyr
  1               5                  10

<210> 7

<211> 17
<212> PRT
<213> Mus musculus

<400> 7
Asp Ile Asp Pro Tyr Asn Gly Asp Thr Ser Tyr Asn Gln Lys Phe Arg
1               5                   10                  15

Asp


<210> 8
<211> 4
<212> PRT
<213> Mus musculus

<400> 8
Gly Leu Arg Phe
1

<210> 9
<211> 237
<212> PRT
<213> Artificial Sequence

<220>
<223> Artificially produced peptide in the form of a single chain
variable region fragment (scFv) which neutralizes IL-18

<400> 9
Glu Ile Gln Leu Gln Gln Ser Gly Pro Glu Leu Val Lys Pro Gly Ala
1               5                   10                  15

Ser Val Lys Val Ser Cys Lys Ala Ser Gly Tyr Ser Phe Thr Asp Tyr
            20                  25                  30

Phe Ile Tyr Trp Val Lys Gln Ser His Gly Lys Ser Leu Glu Trp Ile
        35                  40                  45

Gly Asp Ile Asp Pro Tyr Asn Gly Asp Thr Ser Tyr Asn Gln Lys Phe
    50                  55                  60

Arg Asp Lys Ala Thr Leu Thr Val Asp Gln Ser Ser Thr Thr Ala Phe
65                  70                  75                  80

Met His Leu Asn Ser Leu Thr Ser Glu Asp Ser Ala Val Tyr Phe Cys
                85                  90                  95

Ala Arg Gly Leu Arg Phe Trp Gly Gln Gly Thr Leu Val Thr Val Ser
            100                 105                 110

Ala Gly Gly Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Ala Ser
    130                 135                 140

Leu Gly Glu Arg Val Ser Leu Thr Cys Arg Ala Ser Gln Asp Ile Gly
145                 150                 155                 160

Ser Lys Leu Tyr Trp Leu Gln Gln Glu Pro Asp Gly Thr Phe Lys Arg
                165                 170                 175

Leu Ile Tyr Ala Thr Ser Ser Leu Asp Ser Gly Val Pro Lys Arg Phe
            180                 185                 190

Ser Gly Ser Arg Ser Gly Ser Asp Tyr Ser Leu Thr Ile Ser Ser Leu
        195                 200                 205

Glu Ser Glu Asp Phe Val Asp Tyr Tyr Cys Leu Gln Tyr Ala Ser Ser
    210                 215                 220

Pro Tyr Thr Phe Gly Gly Gly Thr Lys Leu Ala Ile Lys
225                 230                 235

<210> 10
<211> 243
<212> PRT
<213> Artificial Sequence

<220>
<223> Artificially produced peptide in the form of a single chain
variable region fragment (scFv) which neutralizes IL-18

<400> 10
Glu Ile Gln Leu Gln Gln Ser Gly Pro Glu Leu Val Lys Pro Gly Ala
  1               5                  10                  15

Ser Val Lys Val Ser Cys Lys Ala Ser Gly Tyr Ser Phe Thr Asp Tyr
             20                  25                  30

Phe Ile Tyr Trp Val Lys Gln Ser His Gly Lys Ser Leu Glu Trp Ile
         35                  40                  45

Gly Asp Ile Asp Pro Tyr Asn Gly Asp Thr Ser Tyr Asn Gln Lys Phe
     50                  55                  60

Arg Asp Lys Ala Thr Leu Thr Val Asp Gln Ser Ser Thr Thr Ala Phe
 65                  70                  75                  80

Met His Leu Asn Ser Leu Thr Ser Glu Asp Ser Ala Val Tyr Phe Cys
                 85                  90                  95

Ala Arg Gly Leu Arg Phe Trp Gly Gln Gly Thr Leu Val Thr Val Ser
            100                 105                 110

Ala Gly Gly Gly Gly Gly Gly Ser Gly Gly Gly Gly Ser Gly Gly Gly
        115                 120                 125

Gly Ser Asp Ile Gln Met Thr Gln Ser Pro Ser Ser Leu Ser Ala Ser
    130                 135                 140

Leu Gly Glu Arg Val Ser Leu Thr Cys Arg Ala Ser Gln Asp Ile Gly
145                 150                 155                 160

Ser Lys Leu Tyr Trp Leu Gln Gln Glu Pro Asp Gly Thr Phe Lys Arg
                165                 170                 175

Leu Ile Tyr Ala Thr Ser Ser Leu Asp Ser Gly Val Pro Lys Arg Phe
            180                 185                 190

Ser Gly Ser Arg Ser Gly Ser Asp Tyr Ser Leu Thr Ile Ser Ser Leu
        195                 200                 205

Glu Ser Glu Asp Phe Val Asp Tyr Tyr Cys Leu Gln Tyr Ala Ser Ser
    210                 215                 220

Pro Tyr Thr Phe Gly Gly Gly Thr Lys Leu Ala Ile Lys His His His
225                 230                 235                 240

His His His

<210> 11
<211> 324
<212> DNA
<213> Mus musculus

<400> 11
gac atc cag atg acc cag tct cca tcc tcc tta tct gcc tct ctg gga      48
Asp Ile Gln Met Thr Gln Ser Pro Ser Ser Leu Ser Ala Ser Leu Gly
  1               5                  10                  15

gaa aga gtc agt ctc act tgt cgg gca agt cag gac att ggt agt aaa      96
Glu Arg Val Ser Leu Thr Cys Arg Ala Ser Gln Asp Ile Gly Ser Lys
             20                  25                  30

tta tac tgg ctt caa cag gaa cca gat gga act ttt aaa cgc ctg atc     144
Leu Tyr Trp Leu Gln Gln Glu Pro Asp Gly Thr Phe Lys Arg Leu Ile
         35                  40                  45

tac gcc aca tcc agt tta gat tct ggt gtc ccc aag agg ttc agt ggc     192
Tyr Ala Thr Ser Ser Leu Asp Ser Gly Val Pro Lys Arg Phe Ser Gly
     50                  55                  60

agt agg tct ggg tca gat tat tct ctc acc atc agc agc ctt gag tct     240
Ser Arg Ser Gly Ser Asp Tyr Ser Leu Thr Ile Ser Ser Leu Glu Ser
 65                  70                  75                  80

gaa gat ttt gta gac tat tac tgt cta caa tat gct agt tct ccg tac     288
Glu Asp Phe Val Asp Tyr Tyr Cys Leu Gln Tyr Ala Ser Ser Pro Tyr
                 85                  90                  95

acg ttc gga ggg ggg acc aag ctg gca ata aaa cgg                     324
Thr Phe Gly Gly Gly Thr Lys Leu Ala Ile Lys Arg
            100                 105

<210> 12
<211> 339
<212> DNA
<213> Mus musculus

<400> 12
gag atc cag ctg cag cag tct gga cct gag ctg gtg aag cct ggg gct    48
Glu Ile Gln Leu Gln Gln Ser Gly Pro Glu Leu Val Lys Pro Gly Ala
1               5                   10                  15

tca gtg aag gtc tcc tgt aag gct tct ggt tac tca ttc act gac tac    96
Ser Val Lys Val Ser Cys Lys Ala Ser Gly Tyr Ser Phe Thr Asp Tyr
            20                  25                  30

ttc att tac tgg gtg aag cag agc cat gga aag agc ctt gag tgg att    144
Phe Ile Tyr Trp Val Lys Gln Ser His Gly Lys Ser Leu Glu Trp Ile
        35                  40                  45

gga gat att gat cct tat aat ggt gat act agt tac aac cag aag ttc    192
Gly Asp Ile Asp Pro Tyr Asn Gly Asp Thr Ser Tyr Asn Gln Lys Phe
    50                  55                  60

agg gac aag gcc aca ttg act gtt gac cag tcc tcc acc aca gcc ttc    240
Arg Asp Lys Ala Thr Leu Thr Val Asp Gln Ser Ser Thr Thr Ala Phe
65                  70                  75                  80

atg cat ctc aac agc ctg aca tct gag gac tct gca gtc tat ttc tgt    288
Met His Leu Asn Ser Leu Thr Ser Glu Asp Ser Ala Val Tyr Phe Cys
                85                  90                  95

gca aga ggc cta cgg ttc tgg ggc caa ggg act ctg gtc act gtc tct    336
Ala Arg Gly Leu Arg Phe Trp Gly Gln Gly Thr Leu Val Thr Val Ser
            100                 105                 110

gca                                                                339
Ala

<210> 13
<211> 33
<212> DNA
<213> Mus musculus

<400> 13
cgg gca agt cag gac att ggt agt aaa tta tac                        33
Arg Ala Ser Gln Asp Ile Gly Ser Lys Leu Tyr
1               5                   10

<210> 14
<211> 21
<212> DNA
<213> Mus musculus

<400> 14
gcc aca tcc agt tta gat tct                                        21
Ala Thr Ser Ser Leu Asp Ser
1               5

<210> 15
<211> 27
<212> DNA
<213> Mus musculus

<400> 15
cta caa tat gct agt tct ccg tac acg                                27
Leu Gln Tyr Ala Ser Ser Pro Tyr Thr
1               5

<210> 16
<211> 30
<212> DNA
<213> Mus musculus

<400> 16
ggt tac tca ttc act gac tac ttc att tac                            30
Gly Tyr Ser Phe Thr Asp Tyr Phe Ile Tyr
1               5                   10

<210> 17
<211> 51
<212> DNA
<213> Mus musculus

<400> 17
gat att gat cct tat aat ggt gat act agt tac aac cag aag ttc agg    48
Asp Ile Asp Pro Tyr Asn Gly Asp Thr Ser Tyr Asn Gln Lys Phe Arg
1               5                   10                  15

gac                                                                51
Asp

<210> 18
<211> 12
<212> DNA
<213> Mus musculus

<400> 18
ggc cta cgg ttc                                                    12
Gly Leu Arg Phe
1

<210> 19
<211> 711
<212> DNA
<213> Artificial Sequence

<220>
<223> Artificial DNA to code for the amino acid sequence of SEQ
ID NO:9

<400> 19
gag atc cag ctg cag cag tct gga cct gag ctg gtg aag cct ggg gct    48
Glu Ile Gln Leu Gln Gln Ser Gly Pro Glu Leu Val Lys Pro Gly Ala
1               5                   10                  15

tca gtg aag gtc tcc tgt aag gct tct ggt tac tca ttc act gac tac    96
Ser Val Lys Val Ser Cys Lys Ala Ser Gly Tyr Ser Phe Thr Asp Tyr
            20                  25                  30

ttc att tac tgg gtg aag cag agc cat gga aag agc ctt gag tgg att    144

Phe Ile Tyr Trp Val Lys Gln Ser His Gly Lys Ser Leu Glu Trp Ile
        35                  40                  45

gga gat att gat cct tat aat ggt gat act agt tac aac cag aag ttc     192
Gly Asp Ile Asp Pro Tyr Asn Gly Asp Thr Ser Tyr Asn Gln Lys Phe
    50                  55                  60

agg gac aag gcc aca ttg act gtt gac cag tcc tcc acc aca gcc ttc     240
Arg Asp Lys Ala Thr Leu Thr Val Asp Gln Ser Ser Thr Thr Ala Phe
65                  70                  75                  80

atg cat ctc aac agc ctg aca tct gag gac tct gca gtc tat ttc tgt     288
Met His Leu Asn Ser Leu Thr Ser Glu Asp Ser Ala Val Tyr Phe Cys
                85                  90                  95

gca aga ggc cta cgg ttc tgg ggc caa ggg act ctg gtc act gtc tct     336
Ala Arg Gly Leu Arg Phe Trp Gly Gln Gly Thr Leu Val Thr Val Ser
            100                 105                 110

gca ggt gga ggt gga ggc gga tcc ggc gga ggt ggc tct ggc ggt ggc     384
Ala Gly Gly Gly Gly Gly Gly Ser Gly Gly Gly Gly Ser Gly Gly Gly
        115                 120                 125

gga tcg gac atc cag atg acc cag tct cca tcc tcc tta tct gcc tct     432
Gly Ser Asp Ile Gln Met Thr Gln Ser Pro Ser Ser Leu Ser Ala Ser
    130                 135                 140

ctg gga gaa aga gtc agt ctc act tgt cgg gca agt cag gac att ggt     480
Leu Gly Glu Arg Val Ser Leu Thr Cys Arg Ala Ser Gln Asp Ile Gly
145                 150                 155                 160

agt aaa tta tac tgg ctt caa cag gaa cca gat gga act ttt aaa cgc     528
Ser Lys Leu Tyr Trp Leu Gln Gln Glu Pro Asp Gly Thr Phe Lys Arg
                165                 170                 175

ctg atc tac gcc aca tcc agt tta gat tct ggt gtc ccc aag agg ttc     576
Leu Ile Tyr Ala Thr Ser Ser Leu Asp Ser Gly Val Pro Lys Arg Phe
            180                 185                 190

agt ggc agt agg tct ggg tca gat tat tct ctc acc atc agc agc ctt     624
Ser Gly Ser Arg Ser Gly Ser Asp Tyr Ser Leu Thr Ile Ser Ser Leu
        195                 200                 205

gag tct gaa gat ttt gta gac tat tac tgt cta caa tat gct agt tct     672
Glu Ser Glu Asp Phe Val Asp Tyr Tyr Cys Leu Gln Tyr Ala Ser Ser
    210                 215                 220

ccg tac acg ttc gga ggg ggg acc aag ctg gca ata aaa                 711
Pro Tyr Thr Phe Gly Gly Gly Thr Lys Leu Ala Ile Lys
225                 230                 235

<210> 20
<211> 729
<212> DNA
<213> Artificial Sequence

<220>

<223> Artificial DNA to code for the amino acid sequence of SEQ
ID NO:10

<400> 20
gag atc cag ctg cag cag tct gga cct gag ctg gtg aag cct ggg gct     48
Glu Ile Gln Leu Gln Gln Ser Gly Pro Glu Leu Val Lys Pro Gly Ala
1               5                   10                  15

tca gtg aag gtc tcc tgt aag gct tct ggt tac tca ttc act gac tac     96
Ser Val Lys Val Ser Cys Lys Ala Ser Gly Tyr Ser Phe Thr Asp Tyr
            20                  25                  30

ttc att tac tgg gtg aag cag agc cat gga aag agc ctt gag tgg att    144
Phe Ile Tyr Trp Val Lys Gln Ser His Gly Lys Ser Leu Glu Trp Ile
        35                  40                  45

gga gat att gat cct tat aat ggt gat act agt tac aac cag aag ttc    192
Gly Asp Ile Asp Pro Tyr Asn Gly Asp Thr Ser Tyr Asn Gln Lys Phe
    50                  55                  60

agg gac aag gcc aca ttg act gtt gac cag tcc tcc acc aca gcc ttc    240
Arg Asp Lys Ala Thr Leu Thr Val Asp Gln Ser Ser Thr Thr Ala Phe
65                  70                  75                  80

atg cat ctc aac agc ctg aca tct gag gac tct gca gtc tat ttc tgt    288
Met His Leu Asn Ser Leu Thr Ser Glu Asp Ser Ala Val Tyr Phe Cys
                85                  90                  95

gca aga ggc cta cgg ttc tgg ggc caa ggg act ctg gtc act gtc tct    336
Ala Arg Gly Leu Arg Phe Trp Gly Gln Gly Thr Leu Val Thr Val Ser
            100                 105                 110

gca ggt gga ggt gga ggc gga tcc ggc gga ggt ggc tct ggc ggt ggc    384
Ala Gly Gly Gly Gly Gly Gly Ser Gly Gly Gly Gly Ser Gly Gly Gly
        115                 120                 125

gga tcg gac atc cag atg acc cag tct cca tcc tcc tta tct gcc tct    432
Gly Ser Asp Ile Gln Met Thr Gln Ser Pro Ser Ser Leu Ser Ala Ser
    130                 135                 140

ctg gga gaa aga gtc agt ctc act tgt cgg gca agt cag gac att ggt    480
Leu Gly Glu Arg Val Ser Leu Thr Cys Arg Ala Ser Gln Asp Ile Gly
145                 150                 155                 160

agt aaa tta tac tgg ctt caa cag gaa cca gat gga act ttt aaa cgc    528
Ser Lys Leu Tyr Trp Leu Gln Gln Glu Pro Asp Gly Thr Phe Lys Arg
                165                 170                 175

ctg atc tac gcc aca tcc agt tta gat tct ggt gtc ccc aag agg ttc    576
Leu Ile Tyr Ala Thr Ser Ser Leu Asp Ser Gly Val Pro Lys Arg Phe
            180                 185                 190

agt ggc agt agg tct ggg tca gat tat tct ctc acc atc agc agc ctt    624
Ser Gly Ser Arg Ser Gly Ser Asp Tyr Ser Leu Thr Ile Ser Ser Leu
        195                 200                 205

gag tct gaa gat ttt gta gac tat tac tgt cta caa tat gct agt tct    672

Glu Ser Glu Asp Phe Val Asp Tyr Tyr Cys Leu Gln Tyr Ala Ser Ser
    210                 215                 220

ccg tac acg ttc gga ggg ggg acc aag ctg gca ata aaa cat cac cat    720
Pro Tyr Thr Phe Gly Gly Gly Thr Lys Leu Ala Ile Lys His His His
225                 230                 235                 245

cac cat cac                                                        729
His His His

<210> 21
<211> 157
<212> PRT
<213> Homo sapiens

<220>
<221> UNSURE
<222> (73)
<223> "Xaa" means an amino acid of isoleucine or threonine.

<400> 21
Tyr Phe Gly Lys Leu Glu Ser Lys Leu Ser Val Ile Arg Asn Leu Asn
  1               5                  10                  15

Asp Gln Val Leu Phe Ile Asp Gln Gly Asn Arg Pro Leu Phe Glu Asp
             20                  25                  30

Met Thr Asp Ser Asp Cys Arg Asp Asn Ala Pro Arg Thr Ile Phe Ile
         35                  40                  45

Ile Ser Met Tyr Lys Asp Ser Gln Pro Arg Gly Met Ala Val Thr Ile
     50                  55                  60

Ser Val Lys Cys Glu Lys Ile Ser Xaa Leu Ser Cys Glu Asn Lys Ile
 65                  70                  75                  80

Ile Ser Phe Lys Glu Met Asn Pro Pro Asp Asn Ile Lys Asp Thr Lys
                 85                  90                  95

Ser Asp Ile Ile Phe Phe Gln Arg Ser Val Pro Gly His Asp Asn Lys
            100                 105                 110

Met Gln Phe Glu Ser Ser Ser Tyr Glu Gly Tyr Phe Leu Ala Cys Glu
        115                 120                 125

Lys Glu Arg Asp Leu Phe Lys Leu Ile Leu Lys Lys Glu Asp Glu Leu
    130                 135                 140

Gly Asp Arg Ser Ile Met Phe Thr Val Gln Asn Glu Asp
145                 150                 155

<210> 22
<211> 157
<212> PRT
<213> Mus Musculus

<220>
<221> UNSURE
<222> (70)
<223> "Xaa" means an amino acid of methionine or threonine.

<400> 22
Asn Phe Gly Arg Leu His Cys Thr Thr Ala Val Ile Arg Asn Ile Asn
1               5                   10                  15

Asp Gln Val Leu Phe Val Asp Lys Arg Gln Pro Val Phe Glu Asp Met
            20                  25                  30

Thr Asp Ile Asp Gln Ser Ala Ser Glu Pro Gln Thr Arg Leu Ile Ile
        35                  40                  45

Tyr Met Tyr Lys Asp Ser Glu Val Arg Gly Leu Ala Val Thr Leu Ser
    50                  55                  60

Val Lys Asp Ser Lys Xaa Ser Thr Leu Ser Cys Lys Asn Lys Ile Ile
65                  70                  75                  80

Ser Phe Glu Glu Met Asp Pro Pro Glu Asn Ile Asp Asp Ile Gln Ser
                85                  90                  95

Asp Leu Ile Phe Phe Gln Lys Arg Val Pro Gly His Asn Lys Met Glu
            100                 105                 110

Phe Glu Ser Ser Leu Tyr Glu Gly His Phe Leu Ala Cys Gln Lys Glu
        115                 120                 125

Asp Asp Ala Phe Lys Leu Ile Leu Lys Lys Lys Asp Glu Asn Gly Asp
    130                 135                 140

Lys Ser Val Met Phe Thr Leu Thr Asn Leu His Gln Ser
145                 150                 155

<210> 23
<211> 43
<212> DNA
<213> Artificial Sequence

<220>
<223> Designed oligonucleotide as sense primer to amplify a cDNA
fragment coding for an antibody light chain variable region

<400> 23
actagtcgac atgaggrccc ctgctcagwt tyttggmwtc ttg                43

<210> 24
<211> 27
<212> DNA
<213> Artificial Sequence

<220>
<223> Designed oligonucleotide as antisense primer to amplify a cDNA
fragment coding for an antibody light chain variable region

<400> 24
ggatcccggg tggatggtgg gaagatg                                          27

<210> 25
<211> 36
<212> DNA
<213> Artificial Sequence

<220>
<223> Designed oligonucleotide as sense primer to amplify a cDNA
fragment coding for an antibody heavy chain variable region

<400> 25
actagtcgac atggratgga gckggrtctt tmtctt                                36

<210> 26
<211> 28
<212> DNA
<213> Artificial Sequence

<220>
<223> Designed oligonucleotide as antisense primer to amplify a cDNA
fragment coding for an antibody heavy chain variable region

<400> 26
ggatcccggg ccagtggata gacagatg                                         28

<210> 27
<211> 407
<212> DNA
<213> Mus musculus

<220>
<221> CDS
<222> (1)...(407)

<220>
<221> sig peptide
<222> (1)...(60)

<400> 27
atg agg gcc cct gct cag att ttt ggc ttc ttg ttg ctc ttg ttt cca      48
Met Arg Ala Pro Ala Gln Ile Phe Gly Phe Leu Leu Leu Leu Phe Pro
  1               5                  10                  15

ggt acc aga tgt gac atc cag atg acc cag tct cca tcc tcc tta tct      96
Gly Thr Arg Cys Asp Ile Gln Met Thr Gln Ser Pro Ser Ser Leu Ser
             20                  25                  30

gcc tct ctg gga gaa aga gtc agt ctc act tgt cgg gca agt cag gac     144
Ala Ser Leu Gly Glu Arg Val Ser Leu Thr Cys Arg Ala Ser Gln Asp
         35                  40                  45

att ggt agt aaa tta tac tgg ctt caa cag gaa cca gat gga act ttt     192
Ile Gly Ser Lys Leu Tyr Trp Leu Gln Gln Glu Pro Asp Gly Thr Phe
     50                  55                  60

aaa cgc ctg atc tac gcc aca tcc agt tta gat tct ggt gtc ccc aag     240
Lys Arg Leu Ile Tyr Ala Thr Ser Ser Leu Asp Ser Gly Val Pro Lys
65                  70                  75                  80

agg ttc agt ggc agt agg tct ggg tca gat tat tct ctc acc atc agc     288
Arg Phe Ser Gly Ser Arg Ser Gly Ser Asp Tyr Ser Leu Thr Ile Ser
                85                  90                  95

agc ctt gag tct gaa gat ttt gta gac tat tac tgt cta caa tat gct     336
Ser Leu Glu Ser Glu Asp Phe Val Asp Tyr Tyr Cys Leu Gln Tyr Ala
            100                 105                 110

agt tct ccg tac acg ttc gga ggg ggg acc aag ctg gca ata aaa cgg     384
Ser Ser Pro Tyr Thr Phe Gly Gly Gly Thr Lys Leu Ala Ile Lys Arg
        115                 120                 125

gct gat gct gca cca act gta tc                                      407
Ala Asp Ala Ala Pro Thr Val
    130                 135

<210> 28
<211> 412
<212> DNA
<213> Mus musculus

<220>
<221> CDS
<222> (1)...(412)

<220>
<221> sig peptide
<222> (1)...(60)

<400> 28
atg gga tgg agc ggg atc ttt ctc ttc ctc ctg tca gga cct aca ggt      48
Met Gly Trp Ser Gly Ile Phe Leu Phe Leu Leu Ser Gly Pro Thr Gly
  1               5                  10                  15

gtc cac tct gag atc cag ctg cag cag tct gga cct gag ctg gtg aag      96
Val His Ser Glu Ile Gln Leu Gln Gln Ser Gly Pro Glu Leu Val Lys
             20                  25                  30

cct ggg gct tca gtg aag gtc tcc tgt aag gct tct ggt tac tca ttc     144
Pro Gly Ala Ser Val Lys Val Ser Cys Lys Ala Ser Gly Tyr Ser Phe
         35                  40                  45

act gac tac ttc att tac tgg gtg aag cag agc cat gga aag agc ctt     192
Thr Asp Tyr Phe Ile Tyr Trp Val Lys Gln Ser His Gly Lys Ser Leu
     50                  55                  60

gag tgg att gga gat att gat cct tat aat ggt gat act agt tac aac     240
Glu Trp Ile Gly Asp Ile Asp Pro Tyr Asn Gly Asp Thr Ser Tyr Asn
 65                  70                  75                  80

cag aag ttc agg gac aag gcc aca ttg act gtt gac cag tcc tcc acc     288
Gln Lys Phe Arg Asp Lys Ala Thr Leu Thr Val Asp Gln Ser Ser Thr
                 85                  90                  95

aca gcc ttc atg cat ctc aac agc ctg aca tct gag gac tct gca gtc     336
Thr Ala Phe Met His Leu Asn Ser Leu Thr Ser Glu Asp Ser Ala Val
            100                 105                 110

tat ttc tgt gca aga ggc cta cgg ttc tgg ggc caa ggg act ctg gtc     384
Tyr Phe Cys Ala Arg Gly Leu Arg Phe Trp Gly Gln Gly Thr Leu Val
        115                 120                 125

act gtc tct gca gcc aaa acg aca ccc c                               412
Thr Val Ser Ala Ala Lys Thr Thr Pro
    130                 135

<210> 29
<211> 27
<212> DNA
<213> Artificial Sequence

<220>
<223> Designed oligonucleotide as sense primer to amplify a DNA
fragment containing the nucleotide sequence of SEQ ID NO:12

<400> 29
gtcatatgga gatccagctg cagcagt                                        27

<210> 30
<211> 43
<212> DNA
<213> Artificial Sequence

<220>
<223> Designed oligonucleotide as antisense primer to amplify
a DNA fragment containing the nucleotide sequence of SEQ ID NO:12

<400> 30
gaggatccgc ctccacctcc acctgcagag acagtgacca gag                      43

<210> 31
<211> 50
<212> DNA
<213> Artificial Sequence

<220>
<223> Designed oligonucleotide as sense primer to amplify a DNA
fragment containing a part of the nucleotide sequence of SEQ ID
NO:11

<400> 31
tggatccggc ggaggtggct ctggcggtgg cggatcggac atccagatga               50

<210> 32
<211> 31
<212> DNA
<213> Artificial Sequence

<220>
<223> Designed oligonucleotide as antisense primer to amplify
a DNA fragment containing a part of the nucleotide sequence of

SEQ ID NO:11

<400> 32
ccggatcctt attttattgc cagcttggtc c                                    31

<210> 33
<211> 45
<212> DNA
<213> Artificial Sequence

<220>
<223> Designed oligonucleotide as antisense primer to amplify
a DNA fragment containing a part of the nucleotide sequence of
SEQ ID NO:11

<400> 33
tggatcctta gtgatggtga tggtgatgtt ttattgccag cttgg                     45

## Description

This invention relates to a novel biologically active peptide, more particularly, an artificially produced peptide which is capable of neutralizing the biological activities of interleukin-18.

Interleukin-18 (hereinafter abbreviated as "IL-18") is a type of cytokine which mediates signal transduction in immune system. As described in Japanese Patent Kokai Nos.27,189/96 and 193,098/96 and Haruki Okamura et al., "Nature", Vol.378, No.6552, pp.88-91 (1995), IL-18 was provisionally designated "interferon-γ inducing factor" immediately after the discovery. The designation was changed later into "IL-18" in accordance with the proposal in Shimpei Ushio et al., "The Journal of Immunology", Vol.156, pp.4274-4279 (1996). IL-18 in mature form, consisting of 157 amino acids, possesses the properties of inducing in immunocompetent cells the production of interferon-γ (hereinafter abbreviated as "IFN-γ"), a useful biologically active protein, as well as of inducing and enhancing the generation and cytotoxicity of killer cells. Because of the properties, energetic studies are now in progress with the purposes of developing IL-18 as pharmaceuticals such as antiviral, antimicrobial, antitumor and anti-immunopathic agents.

As described above, in nature, cytokines including IL-18 are produced and secreted as substances which are to mediate signal transduction in immune system. Normal immune system secretes cytokines at modulated timing and mediate signals to cells to keep host resistant against harmful substances including viruses, microbes, and tumor cells. When endogenous production or exogenous administration exceeds cytokines beyond normal level in living body, immune system comes into disturbed balance which may affect living body. For example, Masanori Kawashima et al., "Rheumatology in Europe, Journal for Education and Information in Rheumatology", Vol.26, supplement No.2, p.77 (1997) reports elevated IL-18 levels in body fluids of patients with autoimmune diseases, suggesting that there may be a close relationship between the occurrence of inflammatory disorders such as autoimmune diseases and IL-18 production in living body. In order to develop pharmaceuticals which are efficacious in treatment and prevention of the diseases where IL-18 is involved, it is necessary to design pharmaceutically-acceptable substances which are capable of neutralizing the biological activities of IL-18 as well as to establish the mass-production thereof.

In this field, many investigators have been energetically trying to produce or engineer cytokine-neutralizing substances. Hopeful candidates include neutralizing antibodies against cytokines, soluble receptors for cytokines, and cytokine antagonists. The neutralizing antibodies would be more attractive because of their higher specificity and neutralizing activity to target cytokines. However antibodies obtained from non-human mammals exhibit antigenicity when administered in human. Thus the repetitive administration of such antibodies generally does not attain desired efficacy. Such antibodies may cause side effects such as anaphylaxis when administered in human. Although there have been proposed several approaches to solve these problems in antibodies, none of them has been proved to successfully applicable to antibodies in general. There can be found some reports on only a few cytokine-neutralizing substances.

In view of the foregoing, an aim of this invention is to provide a substance which effectively neutralizes the biological activities of IL-18 in mammal including human.
a second aim of this invention is to provide a DNA which codes the substance.
a third aim of this invention is to provide a process of producing the substance.
a fourth aim of this invention is to provide a use of the substance as agent for susceptive diseases.
a fifth aim of this invention is to provide a use of the substance as IL-18 neutralizer.
a sixth aim of this invention is to provide a method to neutralize IL-18 using the substance.
a seventh aim of this invention is to provide a use of the substance as IL-18 inhibitor.
an eighth aim of this invention is to provide a method to inhibit IL-18 using the substance.

The present inventors studied anti-IL-18 antibodies by determining the amino acid sequences for the variable regions, and designed peptides which comprise a part or the whole of the amino acid sequences. The inventors confirmed that the peptides specifically bind to IL-18 and effectively neutralize IL-18. The peptides were also confirmed efficacious in the treatment and prevention of diseases such as immunopathies, inflammatory disorders, and autoimmune diseases which are caused by excessive immunoreaction. In addition the DNAs coding for the peptides were confirmed to facilitate the production of the peptides in desired amounts. This invention is based on these findings.

More particularly, this invention provides an artificially produced peptide which neutralizes a biological activity of IL-18, comprising a part or the whole of the amino acid sequence of variable regions in anti-IL-18 antibody.

This invention also provides a DNA coding for the peptide.

This invention also provides a process of producing the peptide comprising the steps of allowing a DNA coding for the peptide to express and collecting of the expressed peptide.

This invention also provides an agent for susceptive diseases comprising the peptide as effective ingredient.

This invention further provides an IL-18 neutralizer comprising the peptide as effective ingredient.

This invention further provides a method of neutralizing IL-18 by allowing the peptide to act on IL-18.

This invention further provides an IL-18 inhibitor comprising the peptide as effective ingredient.

This invention further provides a method of inhibiting IL-18 by allowing the peptide to act on IL-18.

The invention will now be described in further detail, by way of example only, with reference to the following Examples and accompanying drawings in which:
FIG.1 shows the structure of the recombinant DNA "pEscFv#125 -2H" which contains the nucleotide sequence coding for the peptide of this invention;
FIG.2 shows the neutralizing action of the present peptide on the IL-18 biological activity to induce IFN-y production from immunocompetent cells;
FIG.3 is a half-tone image of SDS-PAGE given on a display showing the specific binding of the present peptide to IL-18;
FIG.4 shows the structure of the recombinant DNA "pEscFv#125-2H.HT" which contains the nucleotide sequence coding for the peptide of this invention; and
FIG.5 shows the neutralizing action of the present peptide on the IL-18 biological activity to induce IFN-y production from immunocompetent cells.

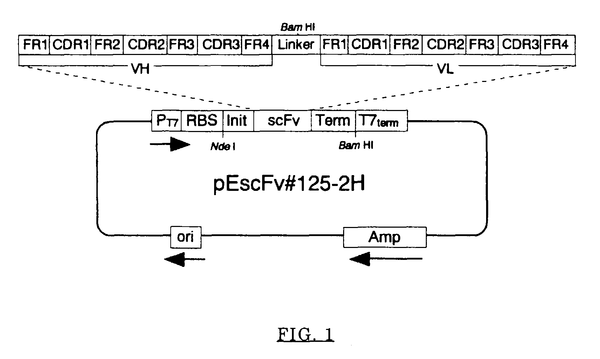

In FIGs. 1 and 4, the symbol P_{T7} represents T7 promotor; RBS, ribosome-binding sequence; Init, initiation codon; scFv, DNA coding for the present peptide; VH, nucleotide sequence coding for the variable region on anti-IL-18 antibody heavy chain; Linker, nucleotide sequence coding for linker sequence; VL, nucleotide sequence coding for the variable region on anti-IL-18 antibody light chain; FR, nucleotide sequence coding for framework structure of anti-IL-18 antibody; His₆, nucleotide sequence coding for the sequence of six residues of histidine; Term, termination codon; ori, replication origin in *Escherichia coli*; Amp, ampicillin-resistant gene; and T7term, T7 terminator.

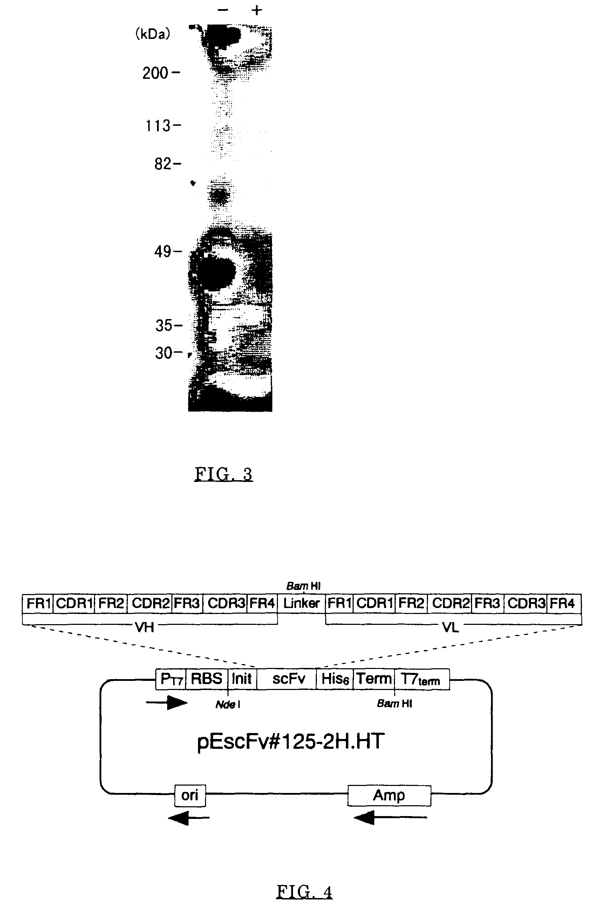

In FIG.3, on lane "-" sample free of non-labeled IL-18 was electrophoresed; and on lane "+", sample with non-labeled IL-18. Left-hand figures indicate the size (kilodaltons, kDa) and electrophoresed position of molecular weight markers.

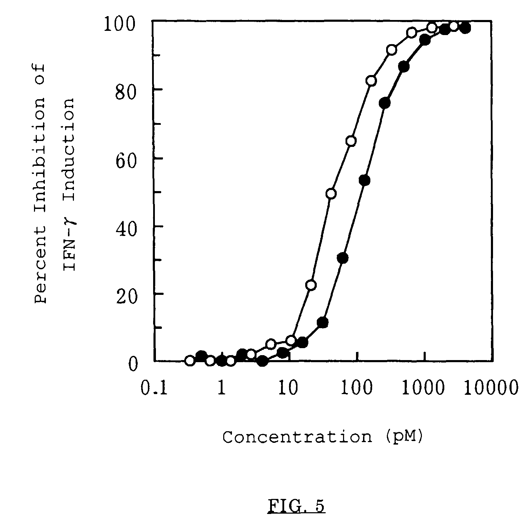

In FIG.5, solid circles (-●-) indicate the results by the present peptide; and hollow circles (-○-), by the anti-IL-18 antibody "#125-2HmAb".

This invention relates to an artificially produced peptide which neutralizes a biological activity of IL-18 (hereinafter, may be described simply with "neutralize(s) IL-18"), comprising a part or the whole of the amino acid sequences of variable regions in anti-IL-18 antibody. The wording "anti-IL-18 antibody" as referred to in this invention means any immunoglobulins which are produced by antibody-producing cells of mammals immunized with IL-18 and capable of recognizing IL-18, regardless of their class and origin and the origin of IL-18 as antigen.

Antibodies generally comprise two light chains and two heavy chains which form a unit by disulfide bonds. The respective chains of antibodies from the same origin conserve certain sequences in C-terminal parts, while are diversified by N-terminal parts consisting of about 110 amino acids. The former parts are called constant regions; and the latter, variable regions. Each variable region consists of particularly divergent parts and relatively-well conserved parts, which are called complementarity-determining regions (hereinafter, abbreviated "CDR(s)") and framework structures, respectively. In each variable region, there exist independent four framework structures and three CDRs which intervene mutually. Specific binding of antibody to antigen involves the variable regions, particularly, CDRs therein. The peptide of this invention comprises a part or the whole of the amino acid sequences of variable regions or six CDRs in anti-IL-18 antibody.

The amino acid sequences of variable regions including CDRs in anti-IL-18 antibodies can be determined as follows usually: RNA is first prepared in conventional manner from anti-IL-18 antibody producing cells. Hybridomas that produce monoclonal antibodies against human or mouse IL-18 are feasible as antibody producing cells. Such hybridoma can be prepared by the methods in Japanese Patent Kokai Nos.217,798/96 and 231,598/96 by the same applicant. Spleen cells, extracted from mammals, usually other than human, pre-immunized with human or mouse IL-18, are also feasible as antibody producing cells. Human IL-18 can be prepared by the methods in Japanese Patent No.2,724,987 by the same applicant; and mouse IL-18, in Japanese Patent Kokai No.27,189/96 by the same applicant. Alternatively human lymphocytes are isolated and stimulated *in vitro* with IL-18 to use as antibody producing cells. Then cDNA for anti-IL-18 antibody is cloned, for example, either by conventional RT-PCR using as template RNA as mentioned above, or screening a cDNA library, preferably a cDNA expression library, constructed from RNA as mentioned above. Construction and screening of such cDNA expression library is detailed, for example, in "Methods in Molecular Biology" edited by S. Paul, Vol.51, pp.355-394, published by Humana press Inc., Totowa, New Jersey, USA (1995). By sequencing the cloned cDNA, the amino acid sequences of the variable regions including CDRs are elucidated. For example, "#125-2HmAb", a type of anti-IL-18 monoclonal antibody, comprises the light chain variable region with the amino acid sequence of SEQ ID NO:1 and the heavy chain variable region with the amino acid sequence of SEQ ID NO:2. The CDRs on the antibody light chain comprise the amino acid sequences of SEQ ID NOs:3-5; and on heavy chain, SEQ ID NOs:6-8.

The peptide of this invention includes the artificially produced peptides which neutralize IL-18 and comprise, as mentioned above, a part or the whole of the amino acid sequences of variable regions in anti-IL-18 antibody, and is distinct from naturally occurring IL-18-neutralizing antibodies of non-human origin. Interleukin-18-neutralizing activity of the present peptide can be detected, for example, by the method described in Example 1-1(a) to test for the inhibitory effect on a biological activity of IL-18, to induce IFN-γ production from immunocompetent cells. The present peptide does not completely contain the amino acid sequences of the constant regions of non-human antibody. The wording "IL-18" as referred to in this invention means a substance that exhibits the biological activities as IL-18, including those in a monomeric form comprising the amino acid sequence as IL-18, for example, shown by SEQ ID NO:22 or 23, multiple form consisting of two or more units comprising such sequence, and complexed form associated with other proteins or substances such as albumin.

Examples of the present peptide are those artificially produced to comprise a part or the whole of the above-mentioned amino acid sequences, of variable regions in anti-IL-18 antibody, if necessary, in combination with desired foreign sequences, more particularly: so-called single chain variable region fragments (hereinafter abbreviated as "scFv") which are engineered by connecting the amino acid sequences of variable regions on heavy and light chains in anti-IL-18 antibody *via* a suitable linker, and humanized monoclonal antibodies or so-called humanized antibodies including chimeric antibodies which are engineered by grafting the amino acid sequences of the variable regions or CDRs therein, if necessary, in combination with some amino acids around the regions, into the corresponding parts of an antibody of human origin. Examples of the present peptide using the amino acid sequences of the monoclonal antibody "#125-2HmAb" are the peptides of SEQ ID NOs:9 and 10 in the form of an scFv, which are engineered by connecting a part or the whole of the variable region sequences in the antibody shown by SEQ ID NOs:1 and 2 *via* a linker sequence composed of glycine and serine, the peptides in the form of a chimeric antibody comprising a part or the whole of the variable region sequences, and the peptides in the form of a humanized antibody comprising a part or the whole of the the CDR sequences in the antibody shown by SEQ ID NOs:3-8. Examples of the present peptide in other form are enzymatic and/or chemical digests of naturally occurring anti-IL-18 neutralizing antibodies, more particularly, antigen-binding fragments (usually designated "Fab") or dimeric forms of the fragments (usually designated "F(ab')₂") obtained by digesting the natural antibodies with proteinase papain or pepsin.

Since the present peptide in the form of an scFv or enzymatically and/or chemically digested antibody neutralizes IL-18 and is substantially deficient in constant regions, which are considered to be involved in antigenicity exhibited in human bodies, the peptide satisfactorily functions even when repeatedly administered to humans. The peptide as an scFv is lowered in molecular weight as compared with the parental antibody, imparted with favorable features including good permeability to tissues in human bodies and productivity in lower costs because it can be easily produced by transformants of microbial hosts. The present peptide in the form of a humanized antibody is substantially human-derived except for the parts involved in the binding to antigen. The peptide, therefore, hardly exhibits antigenicity and satisfactorily neutralizes IL-18 in human bodies. In addition, the peptide as a humanized antibody has a feature distinct from the parental antibody to readily eliminate IL-18 in combination with itself from human bodies, when administered, by the mechanism involving complement system.

The peptide of this invention includes, in addition to the above examples, those further produced from the above-examples by engineering the amino acid sequences with amino acid replacement, addition, and/or deletion in conventional manner as far as they are not substantially deficient in the desired property. For example, it may improve the exemplified peptides in stability to replace one or more cysteines thereof with other ones such as hydrophilic amino acids including glycine and serine and hydrophobic amino acids including alanine and valine or delete the portions including the cysteines. Addition of several residues of histidine to the N- and/or C-termini of the peptides would facilitate the purification thereof while retaining the desired property. To improve the exemplified peptides in physiological actions including pharmaceutical effects, turnover *in vivo,* side effects, antigenicity to humans and IL-18-neutralizing activity, stability, and specificity or affinity to IL-18, it can be conducted to replace up to 30%, more preferably, up to 10% of the total amino acids composing the CDRs with other ones, for example, amino acids similar to the original ones in property or size to original ones. The peptide of this invention includes those thus modified and comprising a part of the CDR sequences. The present peptide also includes those comprising the amino acid sequences of variable regions or CDRs in different two or more anti-IL-18 antibodies as far as they exhibit the desired property, i.e., neutralizing IL-18.

The present peptide is usually prepared by recombinant DNA techniques comprising the steps of artificially expressing the peptide by coding DNA for the peptide and collecting the expressed peptide. This invention also provides the DNA coding for the present peptide and a process of producing the peptide by recombinant DNA techniques, which facilitates the production of the peptide in desired amounts.

The DNA coding for the present peptide is usually obtained by genetic engineering methods from the cDNA obtained through determining the amino acid sequences of anti-IL-18 antibody employed in this invention. In particular, desired sequences in the cDNA, for example, those coding for the variable regions or CDRs, can be connected by conventional PCR methods with foreign nucleotide sequences selected in accordance with the form of the peptide desired. For the peptide in an scFv form, a part or the whole of the nucleotide sequences coding for the variable regions on the antibody light and heavy chains are connected *via* a coding sequence for an appropriate linker, for example, composed of several to several tens of amino acid residues such as serine and glycine. A coding sequence for a desired signal peptide can be further added to the 5'-termini. For the peptide in a chimeric antibody form, the nucleotide sequences coding for a part or the whole of the variable regions on the antibody light and heavy chains are connected with coding sequences for the constant regions on a known human antibody light and heavy chains. For the peptide in a humanized antibody form comprising a part or the whole of human framework structures, coding sequences for the CDRs in the antibody are grafted in coding sequences for a known human antibody to the corresponding parts, if necessary, in combination with coding sequences for several amino acids around the CDRs. The known human antibody employed in this invention is preferable to resemble in three dimensional structure to the parental antibody. Examples of the nucleotide sequences of the present DNA are shown by SEQ ID NO:19, coding for the amino acid sequence of SEQ ID NO:9, and SEQ ID NO:20, coding for SEQ ID NO:10. These examples are obtainable by connecting, using conventional PCR, a part or the whole of the nucleotide sequences of SEQ ID NOs:11 and 12, which are contained by the cDNA from the hybridoma producing the monoclonal antibody "#125-2HmAb", *via* a nucleotide sequence coding for an amino acid sequence composed of glycine and serine. J. S. Huston et al., "Proceedings of the National Academy of Sciences of the United States of America", Vol.85, pp.5879-5883 (1988) describes basic techniques for scFvs, and L. Riechiman et al., "Nature", Vol.332, pp.323-327 (1988), for humanized antibodies including chimeric antibodies.

In this field, it can be conducted, before artificial expression of a DNA coding for a polypeptide, to replace one or more nucleotides of the DNA with different ones or add desired nucleotide sequences to the DNA to improve the efficiency of DNA expression or the property of the expressed polypeptide. The present DNA can be thus modified as far as the desired property does not substantially defect from the resulting peptide. More particularly, the present DNA is modifiable by adding desired restriction enzyme recognition sites, initiation codons, termination codons, promotors, enhancers, etc., to the 5'- and/or 3'-termini. The DNA of this invention includes those coding for the above-mentioned peptides, those complementary to such DNAs, and those with replacement of one or more nucleotides with different ones without changing the amino acid sequences encoded thereby.

The present DNA can be allowed to express in desired hosts of microbial, animal, or plant origin. The present DNA is usually introduced into the hosts in the form of a recombinant DNA. The recombinant DNA, which usually comprises the present DNA and an autonomously replicable vector, can be obtained with less difficulty using conventional recombinant DNA techniques once the desired DNA is available. The vectors into which the present DNA can be inserted are, for example, pET, pKK223-3, pCDNAI/Amp, BCMGSNeo, pcDL-SRa, pKY4, pSV2-neo, pSV-2gpt, pCDM8, pCEV4, pME18S, pEF-BOS, etc. The vectors are preferable to comprise, for example, promotors, enhancers, replication origins, splice sites and/or selection sequences suitable for expression of the present DNA in respective hosts. Using as promotor heat shock protein promotor or interferon-a promotor described in Japanese Patent Kokai No.163,368/95 by the same applicant makes it possible to artificially regulate the present DNA expression in transformants by external stimuli.

The present DNA can be inserted into the vectors by conventional techniques in this field. For example, a gene containing the present DNA and autonomously replicable vector can be first digested with restriction enzymes and/or sonication, and the resulting DNA fragments and vector fragments can be then ligated. Ligation can be facilitated using, in the digestion, restriction enzymes which specifically react on nucleotides such as *Acc*I, *Bam*HI, *Bst*XI, *Eco*RI, *Hin*dIII, *Not*I, *Pst*I, *Sac*I, *Sal*I, *Sma*I, *Spe*I, *Xba*I, and *Xho*I. Ligation can be accomplished by *in vivo* or *in vitro* action of ligase, after annealing of the fragments of the DNA and vector if necessary. The recombinant DNAs thus obtained can unlimitedly replicate in the hosts of microbial, animal or plant origin.

The recombinant DNA can be introduced into desired hosts to produce the present peptide. The hosts feasible in this invention are, for example, conventional cells derived from a desired microbe, plant, vertebrate, or invertebrate, and bodies of a desired animal or plant. The present DNA includes those in the form of a host introduced with the present DNA. To provide the present peptide in lower costs, microbes including *Escherichia coli* and *Bacillus* sp. are preferable for the hosts. For pharmaceutical uses of the present peptide, the hosts of yeast or animal origin are more preferable. Examples of the animal cells for the hosts are 3T3-Swiss albino cells, ATCC CCL-92; C127I cells, ATCC CRL-1616; CHO-K1 cells, ATCC CCL-61; CV-1 cells, ATCC CCL-70; COS-1 cells, ATCC CRL-1650; HeLa cells, ATCC CCL-2; MOP-8 cells, ATCC CRL-1709; and mutants thereof, included by epithelial cells, interstitial cells, or hemopoietic cells derived from a human, monkey, mouse, or hamster. To introduce the present DNA into the hosts, conventional DEAE-dextran method, calcium phosphate method, electroporation method, lipofection method, microinjection methods, and virus infection methods using retroviruses, adenoviruses, herpes viruses, vaccinia viruses, etc., can be arbitrarily employed. To clone the transformant cells which produce the present peptide from the transformation products, the products are cultivated in media, and the resulting cultures are usually examined for the peptide production. The recombinant DNA techniques using mammalian host cells are described in publications such as "Jikken-Igaku-Bessatsu, Saibo-Kogaku Handbook (The Handbook for cell engineering)", edited by Toshio Kuroki, Masaru Taniguchi, and Mitsuo Oshimura, published by Yodosha Co., Ltd., Tokyo, Japan (1992), and "Jikken-Igaku-Bessatsu, Biomanual Series 3, Idenshi-Cloning-Jikken-Ho (The Experimental Manual for Gene Cloning)", edited by Takashi Yokota and Kenichi Arai, published by Yodosha Co., Ltd., Tokyo, Japan (1993).

Once a desired DNA is obtained, so-called transgenic animals and plants introduced with the DNA can be established by conventional methods in this field. The DNA of this invention introduced into a desired host includes those in the form of a transgenic animal or plant. Usual procedures of establishing transgenic animals are briefly described as follows: First, the present DNA is inserted into a desired vector selected based on the species of the host animal to use, if necessary, in combination with desired other DNAs such as promotors and enhancers. The resulting recombinant DNA is introduced into oosperms or embryonic stem cells of the host animal by appropriate methods such as microinjection, electroporation, and infection of viruses with the present DNA. Feasible animals for the hosts are, for example, rodents widely used as experimental animal including mice, rats, and hamsters as well as mammals commonly used as domestic animal including goats, sheep, swine, and bovine because they are easily bred. Next, the DNA-introduced cells are grafted into uterine tubes or uteri of para-pregnant female animals of the same species as the host. Then the newborns delivered spontaneously or by caesarean are screened by hybridization or PCR to select transgenic animals introduced with the present DNA, leading to establishment of the desired trasngenic animals. These procedures for transgenic animals are described in, for example, "Jikken-Igaku-Bessatsu Shin-Idenshikogaku-Handbook (The handbook for genetic engineering)", edited by Masami Muramatsu, Hiroto Okayama, and Tadashi Yamamoto, pp.269-283, published by Yodosha Co., Ltd., Tokyo, Japan (1996).

Procedures of establishing transgenic plants are also conventional in this field. The DNA of this invention can be introduced into plants in usual manner with satisfactory efficiencies, for example, by introduction into plant protoplasts with a vector such as plasmids of the genus *Agrobacterium* including "Ti plasmid" after inserted with the present DNA or by direct injection of metal micro-particles coated with the present DNA into plant bodies or protoplasts using a particle gun. While feasible plants for the hosts are in wide variety, it is preferred from a viewpoint of the safeness in ingestion of the present peptide by humans to use plants for foods such as potatoes, soybeans, wheat, barley, rice, maize, tomatoes, lettuce, alfalfa, apples, peaches, and melons. Then the resulting plant bodies and protoplasts are screened by hybridization or PCR to select ones containing the desired DNA, and in the case of the protoplasts, the selected ones are regenerated into plant bodies, leading to establishment of the desired transgenic plants. "Genetic Engineering", edited by Jane K. Setlow, Vol.16, pp.93-113, published by Plenum Publishing Corporation, New York, USA (1994) gives outlines of the procedures of establishing transgenic plants.

The peptide of this invention can be produced in desired amounts by the process of this invention comprising the steps of allowing the DNA coding for the present peptide to express and collecting the peptide generated by the expression. The present DNA can be allowed to express through cultivation, breeding or planting of the transformant cells, transgenic animals, or transgenic plants, introduced with the present DNA. Media for cultivating the transformant cells can be arbitrarily selected from conventional ones for transformants, which usually contain a buffer and supplemented with inorganic ions such as sodium ion, potassium ion, phosphoric ion, and chloride ion, and in accordance with the metabolite potential of the hosts, microelements, carbon sources, nitrogen sources, amino acids, vitamins, etc., and if necessary, further supplemented with sera, hormones, cell growth factors, cell adhesion factors, etc. Examples of the media are L broth medium, T broth medium, 199 medium, DMEM medium, Ham's F12 medium, IMDM medium, MCDB104 medium, MCDB153 medium, MEM medium, RD medium, RITC80-7 medium, RPMI1630 medium, RPMI1640 medium, WAJC404 medium, etc. The transformant cells can be inoculated to the media in a cell density of 1×10⁴-1×10⁷cells/ml, preferably, 1×10⁵-1×10⁶cells/ml and cultivated at a temperature of about 37°C for one to seven days, preferably, two to four days, in suspension or mono-layer, if necessary, the media can be changed with fresh preparations during the cultivation, to obtain the cultures containing the present peptide. The cultures thus obtained usually contain the present peptide in about 1µg to about 100mg per liter, which may differ dependently on the types of the transformants and cultivation conditions.

To obtain products containing the present peptide from the transgenic animals or plants, desired tissues, organs, or body fluids including bloods, milks, and marrow fluids can be collected after breeding or planting, if necessary, after charging desired external stimuli on the basis of the form of the DNA introduced, for example, the types of the promotors and enhancers contained thereby. The contents of the present peptide in the products are usually about 1ng to about 100µg per one gram by fresh weight.

The obtained cultures or products containing the present peptide can be subjected, if necessary, to cell disruption with sonication, cell-lytic enzymes, and/or surfactants, and the peptide-containing fractions can be separated from the cells or the cell-disruptants by filtration, centrifugation, etc., and then purified to collect the present peptide for use. Conventional techniques to purify biologically active proteins can be arbitrarily employed to the present purification. Examples of the feasible techniques are salting out, dialysis, filtration, separatory sedimentation, ion-exchange chromatography, gel filtration chromatography, absorption chromatography, isoelectric focusing chromatography, hydrophobic chromatography, reversed phase chromatography, affinity chromatography, gel electrophoresis, isoelectric focusing electrophoresis, etc. Fractions separated by such techniques can be tested for the desired properties of the present peptide such as IL-18-neutralizing activity, IL-18-binding activity, molecular weight, and isoelectric point, to purify the peptide by collecting the fractions exhibiting the desired properties. A type of the present peptide which comprises an amino acid sequence having an affinity for a specific substance can be purified by taking advantage of the affinity. For example, the present peptide comprising the sequence of several residues of histidine, which has an affinity for nickel ion, can be easily purified by affinity chromatography using nickel ion immobilized on a water-insoluble carrier. The present peptide, possibly binding to IL-18 with a certain specificity, can be purified well also by affinity chromatography using IL-18 immobilized on a water-insoluble carrier.

The present peptide obtainable as mentioned above neutralizes a biological activity of IL-18. IL-18 is known to exhibit pleiotropic biological activities, as described on the induction of IFN-γ production from immunocompetent cells, induction of killer cell formation, and enhancement of cytotoxicity of killer cells in Japanese Patent No.2,724,987 and Japanese Patent Kokai No.27,189/96 both by the same applicant. In addition, excessive amounts of IL-18 in living bodies may induce inflammation to the bodies. The present peptide is capable of neutralizing the biological activities of IL-18 and suppress inflammation induced in living bodies by IL-18 biological activities.

Because the present peptide is capable of neutralizing the biological activities of IL-18, which activates immune system, the peptide regulates and suppresses immunoreactions and has efficacy in the treatment and prevention of various diseases caused by excessive immunoreactions. Immune system is intrinsically for defending the host body against harmful substances but may cause unfavorable affects to the body by its own functions. For example, when a mammal is grafted with an organ such as kidney, liver, heart, bone marrow, and blood or a tissue such as skins, cornea, vessels, and cardiac valves, rejection reactions or immunoreactions against the alloantigen would induce in the body T cell activation or lymphocyte proliferation which can cause inflammatory disorders. While variable in malignancy, similar phenomena can be also observed in the case of invasion of heteroantigens, which are not recognized as self by host. In autoimmune diseases, inherent components which must be recognized as self induce allergic reactions. Because the present peptide suppresses or regulates immunoreactions as mentioned above in mammalian bodies including humans', the peptide is efficacious in the treatment or prevention of various diseases caused by immunoreactions. The wording "susceptive diseases" as referred to in this invention means, therefore, the diseases caused by excessive immunoreactions and being treated or prevented by the direct or indirect actions of the present peptide. Examples of the susceptive diseases are the rejection reactions relating to grafting organs or tissues, graft-versus-host diseases, hyper-IL-eighteenemia-associated diseases, pernicious anemia, atrophic gastritis, insulin-resistant diabetes, Wegener granulomatosis, discoid lupus erythematodes, ulcerative colitis, cold agglutinin-relating diseases, Goodpasture's syndrome, Crohn's disease, sympathetic ophthalmitis, hyperthyroidism, juvenile onset type diabetes, Sjögren syndrome, autoimmune hepatitis, autoimmune hemolytic anemia, myasthenia gravis, systemic scleroderma, systemic lupus erythematodes, polyleptic cold hemoglobinuria, polymyositis, periarteritis nodosa, multiple sclerosis, Addison's disease, idiopathic thrombocytopenic purpura, Basedow's disease, leukopenia, hemocytophagic syndrome, Behcét's disease, climacterium praecox, rheumatoid arthritis, adult Still's disease, Still's disease, rheumatopyra, chronic thyroiditis, Hodgkin's disease, HIV-infections, asthma, atopic dermatitis, contact dermatitis, allergic nasitis, pollinosis, apitoxin allergy, etc., included by autoimmune diseases, allergic diseases, or immunopathies. The present peptide is also effective in the treatment and prevention of septic shock relating to excessive IFN-γ produced or administered. The present peptide would be further effective in the treatment and prevention of hepatopathies, for example, viral hepatitis, alcoholic hepatitis, toxic hepatitis, primary biliary cirrhosis, fulminant hepatitis, viral hepatocirrhosis, alcoholic hepatocirrhosis, toxic hepatocirrhosis, cholestatic hepatitis, hepatocellular carcinoma, acute hepatitis, fatty liver, tumors of liver, disorders in hepatic vessels, etc., gallbladder disorders or cholepathia, for example, cholangitis, cholecystitis, primary sclerosing cholangitis, gallbladder cancer, cholangioma, etc., pancreatopathies, for example, acute pancreatitis, chronic pancreatitis, pancreatic insufficiency, pancreatic cancer, pancreatic cyst, etc., and nephropathies or glomerular disorders, for example, acute nephritic syndrome, chronic renal failure, renal carcinoma, renal ischemia, renal calculus, glomerulonephritis, glomerulitis, glomerulosclerosis, etc., and additionally effective in alleviating or solving the symptoms associated with these disorders and diseases, for example, anorexia, cenesthopathia, exhaustion, abdominal pain, dorsal pain, jaundice, fever, hepatic encephalopathy, ascites, bleeding tendency, etc. For these disorders and diseases, the present peptide can be used in combination with agents to improve hepatic functions such as protoporphyrin, thiopurine, malotilate, liver hydrolyzates, glycyrrhizin, diisopropylamine dichloroacetate, methylmethionine sulfonium chloride, glutathione, taurine, cianidanol, interferons, vitamin B₁, vitamin B₂, vitamin B₆, vitamin B₁₂, thioctic acid, syo-saiko-to (a Chinese medicine, typically composed of the extracts of *Bupleurum falcatum* Linné, *Pinelliia ternata* Breitenbach, *Zingiber officinale* Roscoe, *Scutellaria baicalensis* Georgi, *Panax ginseng* C.A.Meyer, *Zizyphus jujuba* Miller, and *Glycyrrhiza uralensis* Fisher), dai-saiko-to (a Chinese medicine, typically composed of the extracts of *Bupleurum falcatum* Linné, *Pinelliia ternata* Breitenbach, *Zingiber officinale* Roscoe, *Scutellaria baicalensis* Georgi, *Paeonia lactiflora* Pallas, *Zizyphus jujuba* Miller, *Citrus aurantium* Linné, and *Rheum palmatum* Linné), saiko-keishi-to (a Chinese medicine, typically composed of the extracts of *Bupleurum falcatum* Linné, *Pinelliia ternata* Breitenbach, *Cinnamomum cassia* Blume, *Paeonia lactiflora* Pallas, *Scutellaria baicalensis* Georgi, *Panax ginseng* C.A.Meyer, *Zizyphus jujuba* Miller, *Glycyrrhiza* *uralensis* Fisher, and *Zingiber officinale* Roscoe), aspartic acid, glycyrrhiza, and methionine. In living bodies, IL-18 can enhance Fas ligand production, and Fas ligand can induce IL-18 secretion from cells. Thus the present peptide would be useful in the treatment and prevention of the diseases involving Fas and Fas ligand. Furthermore, the present peptide would be effective in the alleviation or prevention of circulation-system-relating diseases, for example, ischemia, ischemic cardiomyopathy, cerebral ischemia, basilar artery migraine, stroke, aneurysm of brain base, arteriosclerosis, vascular endothelial disorders, diabetes mellitus, occlusion of mesenteric vessel, superior mesenteric artery syndrome, etc., and nerve-system-relating diseases, for example, Parkinson's disease, spinal atrophy, amyotrophic lateral sclerosis, Alzheimer's disease, dementia, cerebrovascular dementia, AIDS dementia, encephalomyelitis, etc.

Thus the agent for the susceptive diseases comprising the present peptide as an effective ingredient has a variety of uses, for example, as an anti-autoimmune disease agent, anti-allergy agent, anti-inflammation agent, immunosuppressant, hemopoietic agent, leukocytopoietic agent, antalgic, antipyretic, hepatic-function-improving agent, etc. While variable dependently on the forms of the agent and the types and symptoms of the susceptive diseases, the present agent is usually prepared to contain the present peptide in a concentration of 0.00001-100%(w/w), preferably, 0.0001-20%(w/w) on a dry solid basis in the form of a liquid, suspension, paste, or solid.

The present agent for the susceptive diseases includes those in the form of the present peptide alone and the form of compositions, for example, with one or more of physiologically acceptable carriers, excipients, diluents, adjuvants, stabilizers, and if necessary, other biologically active substances. Examples of the stabilizers are proteins including serum albumins and gelatin, saccharides including glucose, sucrose, lactose, maltitol, trehalose, sorbitol, maltitol, mannitol, and lactitol, buffers involving succinate or phosphate, etc. Examples of the biologically active substances feasible in the present agent are FK506, glucocorticoid, cyclophosphamide, nitrogen mustard, triethylenethiophosphoramide, busulfan, pheniramine mustard, chlorambucil, azathioprine, 6-mercaptopurine, 6-thioguanine, 6-azaguanine, 8-azaguanine, 5-fluorouracil, cytarabine, methotrexate, aminopterin, mitomycin C, daunorubicin hydrochloride, actinomycin D, chromomycin A3, bleomycin hydrochloride, doxorubicin hydrochloride, cyclosporin A, L-asparaginase, vincristine, vinblastine, hydroxyurea, procarbazine hydrochloride, adrenocortical hormone, auri colloid, receptor antagonists and neutralizers for cytokines other than IL-18 including antibodies against interleukin-1 receptor proteins, interleukin-2 receptor proteins, interleukin-5 receptor proteins, interleukin-6 receptor proteins, interleukin-8 receptor proteins and interleukin-12 receptor proteins, respectively, and antagonists for TNF-α receptors, TNF-β receptors, interleukin-1 receptors, interleukin-5 receptors and interleukin-8 receptors, respectively, etc.

The present agent for the susceptive diseases includes pharmaceuticals in a minimal dose unit form, for example, those containing the present peptide in an amount corresponding to a single dose or its multiple (up to 4-fold) or divisor (1/40 or more) dose, and can be prepared in physically united forms suitable for administration. Examples of the pharmaceuticals are an injection, liquid, powder, granule, syrup, tablet, capsule, external agent, etc. The present agent can be administered effectively both through peroral and non-peroral routes to treat and prevent the susceptive diseases. A dose of the agent for a patient with the susceptive diseases can be determined from an endogenous IL-18 level of the patient. The endogenous IL-18 level can be measured, for example, by applying the detection method in Japanese Patent Kokai No.231,598/96 by the same applicant or the diagnostic method in Japanese Patent Kokai No.96,730/98 by the same applicant to biological samples from the patient such as blood, bone marrow fluid, and arthrosis fluid. By comparing with a standard level measured similarly with normal samples, the excessive amount of IL-18 in the patient can be estimated. The dose for the patient can be set to contain the present peptide in an amount sufficient to neutralize the excessive IL-18 estimated. While a sufficient amount for the present peptide to neutralize IL-18 might vary dependently on the form of the peptide or administration routes of the agent, the amount is usually 1/2-fold or higher to IL-18 on a molar basis. In accordance with the dose thus determined, the present agent can be administered to the patient at least one shot through peroral route or non-peroral routes such as intradermal, subcutaneous, intramuscular, and intravenous routes with respect to the types or symptoms of the susceptive diseases, the sites where excessive IL-18 was observed, etc. The present agent is usually administered, to an adult human patient, in a dose of 1µg-1g/shot, more preferably, about 10µg-100mg/shot on the present peptide basis with a frequency of 1-4shot/day or 1-5shot/week over one day to one year.

The DNA coding for the present peptide is also useful in so-called gene therapy. In conventional manner for gene therapy, the present DNA is inserted into a vector derived from virus including retroviruses, adenoviruses, and adeno-associated viruses or incorporated in a liposome such as cationic polymers and membrane-fusible liposomes and then injected into patients with diseases caused by excessive endogenous IL-18, or the DNA is introduced *in vitro* into lymphocytes collected from the patients and injected by autografting the cells. In adoptive immuno gene therapies, introducing the DNA of this invention into effector cells similarly as in the above manner can enhance the cytotoxicity of the effector cells against tumors and virus-infected cells, leading to intensification of adoptive immunotherapy. In tumor vaccine gene therapy, tumor cells extracted from patients are introduced with the present DNA similarly as in the above manner for gene therapy, proliferated *in vitro* to a prescribed level, and then autografted. The autografted tumor cells can act as vaccine in the patients, exhibiting intense and antigen-specific antitumor immunity. Thus the present DNA exhibits a remarkable efficacy in gene therapy for various diseases, for example, malignant tumors, vial diseases, infections and autoimmune diseases, as well as in suppression of rejection reaction and excessive immunoreaction relating to grafting organs and allergic diseases. General procedures for gene therapies are detailed in "Jikken-Igaku-Bessatsu, Biomanual U P Series, Idenshichiryo-no-Kisogijutsu (Basic techniques for the gene therapy)", edited by Takashi Shimada, Izumi Saito, and Keiya Ozawa, published by Yodosha Co., Ltd., Tokyo, Japan (1996).

The present peptide, possessing the properties of IL-18 recognition, binding, neutralization, and inhibition, is used as the effective ingredient of IL-18 neutralizer and inhibitor of this invention as well as in IL-18 neutralization and inhibition methods of this invention. These agents and methods are efficacious in the treatment of various diseases caused by excessive IL-18 produced or administered. The present peptide is also useful in affinity chromatography and label assay to purify and detect IL-18. In addition, the present peptide is useful in *in vivo* and *in vitro* screening for agonists and antagonists to IL-18.

The followings explain this invention with Examples. The techniques employed in Examples 1-3 are conventional in this field, as described in detail in "Jikken-Igaku-Bessatsu, Saibo-Kogaku Handbook (The Handbook for cell engineering)", edited by Toshio Kuroki, Masaru Taniguchi, and Mitsuo Oshimura, published by Yodosha Co., Ltd., Tokyo, Japan (1992), and "Jikken-Igaku-Bessatsu, Biomanual Series 3, Idenshi-Cloning-Jikken-Ho (The Experimental Manual for Gene Cloning)", edited by Takashi Yokota and Kenichi Arai, published by Yodosha Co., Ltd., Tokyo, Japan (1993). This invention should not be restricted to the Examples:

### Example 1

### Peptide and DNA coding for the peptide

### Example 1-1

### Selection of anti-IL-18 antibody

### Example 1-1(a)

### Selection of anti-IL-18 antibody-producing hybridoma

A polypeptide having the amino acid sequence of SEQ ID NO:21 was prepared as human IL-18 in accordance with the process for producing polypeptide in Japanese Patent No.2,724,987 by the same applicant. BALB/c mice were immunized with the polypeptide, and spleen cells were prepared from the immunized mice, in accordance with the method in Japanese Patent Kokai No.231,598/96 by the same applicant. The spleen cells were subjected to fusing reaction with Sp2/0-Ag14 cells, ATCC CRL-1581, derived from mouse myeloma, in accordance with the method in Japanese Patent Kokai No.231,598/96 to generate hybridomas. The hybridomas were appropriately divided into wells of microplates and cultivated in usual manner at 37°C for a week.

In accordance with the method in Japanese Patent Kokai No.231,598/96 by the same applicant, the culture supernatants were examined for the reactivity with human IL-18 by enzyme immunoassay, and hybridomas that produced the reactive supernatants were subjected to limit dilution, resulting in cloning several hybridomas that produce anti-IL-18 antibodies.

The cloned hybridomas were cultivated in respective wells of a 96-well microplate in usual manner, and the supernatants were examined for IL-18-neutralizing activity by a test for the inhibitory effect of a sample on the IL-18 biological activity to induce IFN-γ production from immunocompetent cells. For the immunocompetent cells, KG-1 cells, ATCC CCL-246, derived from a bone marrow cell of a patient with human acute myelogenous leukemia, were used, and the culture supernatants of hybridomas were diluted to use for the test samples in desired various ratios with RPMI1640 medium (pH7.4) supplemented with 10%(v/v) fetal calf serum.

KG-1 cells were proliferated in usual manner to give desired cell numbers, and the cells were suspended in RPMI1640 medium (pH7.4) supplemented with 10%(v/v) fetal calf serum to give a cell density of 2×10⁶cells/ml. The cell suspension was distributed to the wells of 96-well microplates in a volume of 0.1ml/well. In parallel, human IL-18 was prepared in a 5ng/ml solution, and 0.05ml of the solution was mixed with 0.05ml of any one of the test samples or, for control, RPMI1640 medium (pH7.4) supplemented with 10%(v/v) fetal calf serum. The mixtures were added to the wells with KG-1 cells, and the microplates were incubated at 37°C for 24 hours in a 5%(v/v) CO₂ incubator. From the wells the culture supernatants were collected and assayed on produced IFN-γ by conventional enzyme-linked immuno solvent assay using a human IFN-γ standard, Gg23-901-530, available from National Institute of Health, USA. Culture supernatants of some hybridomas effectively and dose-dependently inhibited the IL-18 biological activity to induce IFN-γ production observed in control. A hybridoma that exhibited the most strong inhibition was selected and named "#125-2H".

### Example 1-1(b)

### Preparation of anti-IL-18 antibody

The hybridoma "#125-2H", selected in Example 1-1(a), was proliferated intraperitoneally of BALB/c mice in accordance with the method in Japanese Patent Kokai No.231,598/96 by the same applicant. Ascites was collected from the mice, and the monoclonal antibody produced by the hybridoma "#125-2H" was collected from the ascites in accordance with the method in Japanese Patent Kokai No.231,598/96 by the same applicant. Conventional analysis revealed the monoclonal antibody belongs to the class of IgG₁. The monoclonal antibody effectively and dose-dependently inhibited the IL-18 biological activity to induce IFN-γ production from KG-1 cells, when examined by the test in Example 1-1(a), confirming that the antibody is a type of IL-18-neutralizing antibody. The monoclonal antibody was named "#125-2HmAb".

### Example 1-2

### Amino acid sequences for variable regions of anti-IL-18 antibody

The hybridoma "#125-2H", obtained in Example 1-1(a), was suspended in RPMI1640 medium (pH7.4) supplemented with 10%(v/v) fetal calf serum and cultivated in a 5%(v/v) CO₂ incubator while scaling up. After the cell density reached desired level, the cells were transferred to micro-reaction tubes, washed thrice with phosphate-buffered saline (hereinafter abbreviated as "PBS"), and suspended in a fresh preparation of PBS. The cell suspension was transferred to fresh micro-reaction tubes in 5×10⁶cells/tube, and admixed with 1.0ml/tube RNA preparation reagent "ULTRASPEC LS II", commercialized by BIOTEX LABORATORIES Inc., Edmonton, Canada. The mixture was further admixed with 0.2ml/tube chloroform, stirred for 15 seconds, and allowed to stand on ice for five minutes. After the tubes were centrifuged, the upper phases were collected, pooled, admixed with the equal volume of 2-propanol, and allowed to stand on ice for five minutes. After the resulting mixture was centrifuged and the supernatant was removed, the precipitate was washed twice with 75%(v/v) ethanol aqueous solution, dried *in vacuo,* and dissolved in sterilized-distilled water to obtain the total RNA fraction of "#125-2H". A portion of the fraction was examined for the absorbance at 260nm to estimate the RNA content.

The obtained total RNA was placed in two micro-reaction tubes to give 1.0µg/tube, and sterilized-distilled water was added to give a final volume of 10.1µl each. After the tubes were allowed to stand at 70°C for five minutes and then cooled on ice, reverse transferase reaction was conducted in usual manner. In respective tubes, the reaction volume was set at 20µl, and the reaction mixture was set to contain 5mM MgCl₂, 10mM Tris-HCl buffer (pH8.3), 50mM KCl, 1.25mM dNTP mix, 0.01µg/µl random-hexa-deoxyribonucleotide, 2mM dithiothreitol, 0.875unit/µl RNase inhibitor, and 10unit/µl reverse transferase. The temperatures were controlled at 25°C for 10 minutes, at 42°C for 30 minutes, and at 99°C for five minutes in this order, and then cooled to 4°C.

The two tubes of reverse transferase reaction product were individually used as template to conduct two lines of PCR: one, called "PCR A", for amplifying a cDNA fragment coding for the variable region on the antibody light chain; and the other, called "PCR B", the antibody heavy chain. Oligonucleotides as PCR primer were designed by referencing the primers in S. Tarran Jones, "Bio/Technology", Vol.9, pp.88-89 (1991) and prepared in usual manner. SEQ ID NO:23 shows the sequence of the oligonucleotide as sense primer for PCR A; and SEQ ID NO:25, for PCR B. SEQ ID NO:24 shows the sequence of the oligonucleotide as antisense primer for PCR A; and SEQ ID NO:26, for PCR B. Both for PCRs A and B, the reaction mixture was set to give a volume of 100µl and to contain 100mM KCl, 10mM (NH₄)₂SO₄, 20mM Tris-HCl buffer (pH8.8), 2mM MgCl₂, 0.01%(w/v) non-ionic surfactant "TRITON X-100", 10µg/ml bovine serum albumin, 0.125mM dNTP mix, appropriate amounts of sense and antisense primers, and 0.025unit/µl Pfu DNA polymerase, commercialized by STRATAGENE CLONING SYSTEMS, La Jolla, California, USA. The temperatures were controlled under 40 cycles of incubations at 94°C for one minute, at 60°C for one minute, and at 72°C for one minute in this order both for PCRs A and B.

From the respective PCR products, amplified cDNAs were collected by polyethylene glycol precipitation and subjected to ligation reaction with plasmid vector "pCR-SCRIPT CAM SK(+)" using cloning kit "pCR-SCRIPT CAM SK(+) CLONING KIT", commercialized by STRATAGENE CLONING SYSTEMS, La Jolla, California, USA, in accordance with the accompanying instructions. With a portion of each reaction product, competent cells of *Escherichia coli* strain "XL1-BLUE MRF' KAN", commercialized by STRATAGENE CLONING SYSTEMS, La Jolla, California, USA, were transformed in accordance with the accompanying instructions. The transformed *Escherichia coli* cells were inoculated to L-agar plate medium containing 30µg/ml chloramphenicol and cultivated at 37°C overnight under standing conditions. The formed colonies were inoculated to L-broth medium containing 30µg/ml chloramphenicol and cultivated at 37°C overnight under shaking conditions. From the resulting cultures cells were collected, and from the cells recombinant DNAs were collected in usual manner. The recombinant DNAs were sequenced by conventional dideoxy method. The recombinant DNA derived from PCR A contained the nucleotide sequence of SEQ ID NO:27; and the recombinant DNA from PCR B, SEQ ID NO:28. These nucleotide sequences coded for the amino acid sequences aligned therewith.

Variable regions on the light and heavy chains of antibodies commonly have a structure that consists of four types of framework structures and three types of CDRs which intervene mutually. Antibodies of the same origin relatively well conserve amino acid sequences in the framework structures but are diversified by the CDR sequences. By taking advantage of the features, the above-determined amino acid sequences were compared with reported sequences of variable regions in mouse antibodies to try to elucidate the monoclonal antibody "#125-2HmAb" on amino acid sequences of the variable regions on both chains and the CDRs therein. As a result, the monoclonal antibody was concluded to have a sequence of the amino acids 21-128 of the amino acid sequence aligned with SEQ ID NO:27 for the light chain variable region, also shown by SEQ ID NO:1, and a sequence of the amino acids 20-132 of the amino acid sequence aligned with SEQ ID NO:28 for the heavy chain variable region, also shown by SEQ ID NO:2. The three types of the CDRs on the monoclonal antibody light chain were concluded to contain the amino acid sequences of SEQ ID NOs:3-5; and the three CDRs on the heavy chain, SEQ ID NOs:6-8. SEQ ID NOs:11-18 show the nucleotide sequences from the hybridoma "#125-2H" coding for these amino acid sequences of SEQ ID NOs:1-8, respectively.

### Example 1-3

### Peptide and DNA coding for the peptide

### Example 1-3(a)

### Preparation of DNA, recombinant DNA, and transformant

A DNA coding for an scFv peptide comprising a part or the whole of the amino acid sequences of SEQ ID NOs:1 and 2, which are of the variable regions on light and heavy chains of the monoclonal antibody "#125-2H", was prepared by combining conventional genetic engineering methods as follows: Total RNA was first prepared from the hybridoma "#125-2H" and subjected to reverse-transferase reaction in two micro-reaction tubes in accordance with the methods in Example 1-2. The two tubes of the reaction product were then used as template to conduct two lines of PCR: one, called "PCR C", for amplifying a DNA fragment containing the nucleotide sequence of SEQ ID NO:12; and the other, called "PCR D", for amplifying a DNA fragment containing a part of SEQ ID NO:11. Oligonucleotides as primer for these lines of PCR were designed on the basis of the sequence determined in Example 1-2 and prepared in usual manner. SEQ ID NO:29 shows the sequence of the oligonucleotide as sense primer for PCR C; and SEQ ID NO:31, for PCR D. SEQ ID NO:30 shows the sequence of the oligonucleotide as antisense primer for PCR C; and SEQ ID NO:32, for PCR D. The compositions of reaction mixtures both for PCRs C and D were set to correspond to the case of PCR A in Example 1-3. The temperatures were controlled under 3 cycles of incubations at 94°C for one minute, at 35°C for one minute, and at 72°C for one minute in this order, followed by 32 cycles of incubations at 94°C for one minute, at 60°C for 45 seconds, and at 72°C for one minute in this order, and then at 4°C.

Correspondingly to Example 1-2, collection of amplified DNA from the PCR products, ligation reaction of the collected DNA with plasmid vector "pCR-SCRIPT CAM SK(+)", transformation of *Escherichia coli* with the ligation products, cultivation of the transformants, and collection of recombinant DNAs from the culture were carried out. Analysis by dideoxy method confirmed that the recombinant DNA from PCR C contains the nucleotide sequence of SEQ ID NO:12; and the recombinant DNA from PCR D, a part of SEQ ID NO:11.

Restriction enzyme digestion was applied to the recombinant DNA from PCR C with *Nde*I and *Bam*HI, the recombinant DNA from PCR D with *Bam*HI, and plasmid vector "pET-3a", commercialized by TOYOBO Co., Osaka, Japan, with *Nde*I and *Bam*HI. Appropriate amounts of the digests were placed in a micro-reaction tube, and the mixture was subjected to ligation reaction using "LIGATION KIT VERSION 2", commercialized by TAKARA SHUZO Co., Ltd., Ohtsu, Shiga, Japan, in accordance with the accompanying instructions. Competent cells of *Escherichia coli* strain "JM109", commercialized by TAKARA SHUZO Co., Ltd., Ohtsu, Shiga, Japan, were transformed in usual manner with the ligation product, the transformants were cultivated, and a recombinant DNA was collected from the cultures. Analysis by dideoxy method confirmed that the recombinant DNA contains the nucleotide sequence of SEQ ID NO:19 coding for the amino acid sequence of SEQ ID NO:9. Thus-obtained recombinant DNA was named "pEscFv#125-2H". The amino acid sequence of SEQ ID NO:9 consists of the amino acid sequence of SEQ ID NO:2 for the heavy chain variable region in the monoclonal antibody "#125-2HmAb", that for a linker composed of glycine and serine, and a part of SEQ ID NO:1 for the light chain variable region in the antibody, which are positioned in this order from the N-terminus. As shown in FIG.1, the recombinant DNA "pEscFv#125-2H" orderly contains an initiation codon, the amino acid sequence of SEQ ID NO:19, and a termination codon downstream of T7 promotor and ribosome binding sequence. With the recombinant DNA, competent cells of *Escherichia coli* strain "BL21(DE3)pLysS", commercialized by TOYOBO Co., Osaka, Japan, were transformed. The resulting transformant was named "EscFv#125-2H".

### Example 1-3(b)

### Production of peptide by transformant

The transformant "EscFv#125-2H", obtained in Example 1-3(b), was pre-cultivated in usual manner in L-broth medium at 37°C under shaking conditions overnight, and lml of the pre-culture was inoculated into 100ml of T-broth medium containing 50µg/ml ampicillin and 30µg/ml chloramphenicol prepared in a 500ml-Erlenmeyer flask. The medium was subjected to cultivation at 37°C under shaking conditions while the culture was being monitored for the absorbance at 600nm through a 1cm-width cell. When the absorbance reached a value of about 0.5, isopropyl-β-D-thiogalactopyranoside (hereinafter abbreviated as "IPTG") was added to the culture to give a final concentration of 0.4mM, and cultivation was continued for further five hours.

The resulting culture was centrifuged to collect cells, and the cells were frozen at -80°C. The cells were, after thawing, suspended in 0.01M Tris buffer (pH8.0) containing 0.5M urea and 0.1M NaH₂PO₄ (hereinafter called "0.5M urea solution") and disrupted by sonication followed by shaking for one hour. From the cell disruptants, insoluble components were collected by centrifugation to obtain the inclusion body fraction. The inclusion body fraction was suspended in 0.5M urea solution, sonicated, and washed with 0.5M urea solution to obtain the washed inclusion body fraction. The washed inclusion body fraction was solubilized with 0.01M Tris buffer (pH8.0) containing 6.0M urea and 0.1M Na₂HPO₄. The solubilized product was clarified by centrifugation from insoluble components and resolved on gel filtration using "SUPERDEX 75HR10/30", commercialized by AMERSHAM PHARMACIA BIOTECH KK, Tokyo, Japan, as carrier and PBS as eluent to collect the void fraction eluted. The collected fraction was repeatedly subjected to dialysis against PBS containing 8.0M urea to denature the proteinaceous components and reduction of urea concentration in the dialyzing solution to renature the denatured proteinaceous components. The dialyzed product was resolved on gel filtration similarly as above, and a fraction corresponding to molecular weights of about 25-30kDa was collected. The collected fraction was about 2ml and contained about 100µg/ml protein. Analysis by conventional sodium dodesyl sulfate-polyacrylamide gel electrophoresis (hereinafter abbreviated as "SDS-PAGE") revealed that the collected fraction contained a peptide with a molecular weight of about 29kDa in a purity of about 95% or higher.

### Example 1-3(c)

### Neutralization of IL-18 biological activity by peptide

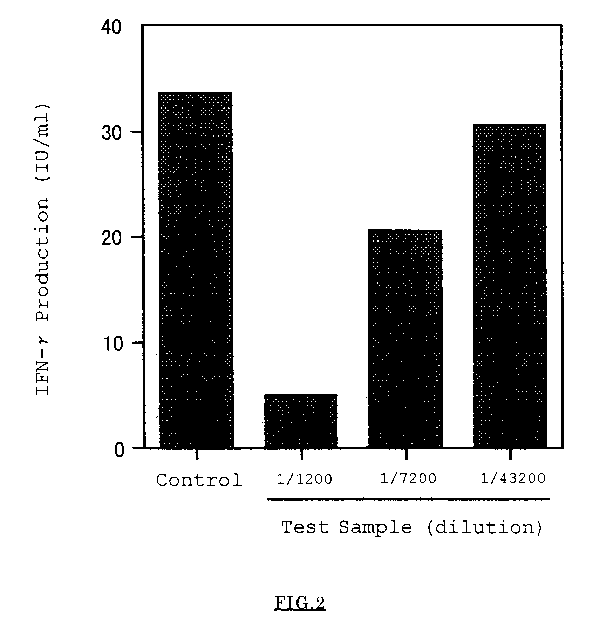

The peptide-containing fraction was diluted 1/1200, 1/7200, and 1/43200-fold with RPMI1640 medium supplemented with 10%(v/v) fetal calf serum, and the dilutions were examined for by the test in Example 1-1(a) IL-18-neutralizing activity. The results are in FIG.2.

As shown in FIG.2, the peptide-containing fraction of Example 1-3(b) dose-dependently inhibited the IL-18 biological activity to induce IFN-γ production from KG-1 cells observed in control. The molecular weight of the peptide estimated by SDS-PAGE well corresponded to the calculated molecular weight of the amino acid sequence of SEQ ID NO:9, about 25kDa. The results of Examples 1-1 to 1-3 indicate that the peptide of Example 1-3(b) is a type of the peptide of this invention, having the amino acid sequence of SEQ ID NO:9, an artificially produced peptide which neutralizes a biological activity of IL-18 and contains a part or the whole of the amino acid sequences of SEQ ID NOs:1 and 2, of the variable regions in anti-IL-18 antibody. These results also indicate that the DNA obtained in Example 1-3(a) is a type of the DNA of this invention, coding for the present peptide, and the DNA facilitates the production of the peptide by the process using the DNA, as shown in Example 1-3(b).

### Example 1-3(d)

### Specific binding of peptide to IL-18

Human IL-18 prepared similarly as in Example 1-1(a) was labelled with ¹²⁵I in usual manner and diluted with PBS containing 0.1%(w/v) bovine serum albumin (hereinafter called "BSA/PBS") into an 8ng/µl ¹²⁵I-labelled human IL-18 solution. The solution was placed in a volume of 0.5µl/tube in two micro-reaction tubes, and to each tube 6.5µl of the gel-filtrated fraction of Example 1-3(b) corresponding to about 25-30kDa, containing the present peptide. To one of the tubes 3µl of BSA/PBS was further added, and to the other the same volume of BSA/PBS containing 3µg of non-labelled human IL-18 were further added. The tubes were shaken at 4°C for one hour. To each tube 4mM aqueous solution of polymerizing agent "BS³" commercialized by PIERCE CHEMICAL Co., Rockford, USA, was added in a volume of 0.5µl, and the tubes were allowed to stand on ice for 30 minutes to effect polymerizing reaction. The reaction was terminated by adding 0.5µl of 1M Tris buffer (pH7.5) per tube and allowing to stand for 15 minutes. The reaction products were subjected along with molecular weight markers to conventional SDS-PAGE using DTT as reducing agent, and the gel was subjected to autoradiography in usual manner. The results are in FIG.3.

As shown in FIG.3, on lane "-", the system free of non-labelled IL-18 exhibited a remarkable band at a molecular weight of about 44kDa. This indicates that the peptide of Example 1-3(b), with the calculated molecular weight of about 25kDa, bound to ¹²⁵I-labelled human IL-18 with the calculated molecular weight of about 18kDa in a molar ratio of about one to one. As shown in FIG.3, on lane "+", the band was diminished by the addition of non-labelled human IL-18, indicating that the binding is specific. The results of Examples 1-3(d) and 1-3(c) indicate that the present peptide specifically binds to IL-18 to neutralize the biological activities possibly by inhibiting the binding of IL-18 to its specific receptor on cells.

### Example 2

### Peptide and DNA coding for the peptide

### Example 2-1

### Preparation of DNA, recombinant DNA, and transformant

A line of PCR, called "PCR E", was carried out under the same conditions as PCR D in Example 1-3(a) except for using the oligonucleotide of SEQ ID NO:33 prepared in usual manner as antisense primer. In parallel, another line of PCR was carried out under the same conditions as PCR C in Example 1-3(a).

Correspondingly to Example 1-3(a), collection of amplified DNAs from the PCR products, ligation of the collected DNAs with plasmid vector "pCR-SCRIPT CAM SK(+)", transformation of *Escherichia coli* with the ligation products, cultivation of the transformants, and collection of recombinant DNAs from the cultures were carried out. Analysis by dideoxy method confirmed that the recombinant DNA from PCR C contains the nucleotide sequence of SEQ ID NO:12; and the recombinant DNA from PCR E, a part of the nucleotide sequence of SEQ ID NO:11. Restriction enzyme digestion was applied to the recombinant DNA from PCR C with *Nde*I and *Bam*HI, the recombinant DNA from PCR E with *Bam*HI, and plasmid vector "pET-3a", employed in Example 1-3(a), with BamHI. Appropriate amounts of these digests were placed in a micro-reaction tube and subjected to ligation reaction using "LIGATION KIT VERSION 2", commercialized by TAKARA SHUZO Co., Ltd., Ohtsu, Shiga, Japan, in accordance with the accompanying instructions. By conventional methods, transformation of competent cells of *Escherichia coli* strain "JM109", commercialized by TAKARA SHUZO Co., Ltd., Ohtsu, Shiga, Japan, with the ligation product, cultivation of the transformants, and collection of a recombinant DNA from the cultures were carried out. Analysis by dideoxy method confirmed that the recombinant DNA contains the nucleotide sequence of SEQ ID NO:20 coding for the amino acid sequence of SEQ ID NO:10 and named "pEscFv#125-2H.HT". The amino acid sequence of SEQ ID NO:10 consists of the amino acid sequences of SEQ ID NO:2 for the heavy chain variable region in the monoclonal antibody "#125-2HmAb", a linker composed of glycine and serine, a part of SEQ ID NO:1 for the light chain variable region in the antibody, and six residues of histidine, which are positioned in this order from the N-terminus. As shown in FIG.4, the recombinant DNA "pEscFv#125-2H" orderly contained an initiation codon, the nucleotide sequence of SEQ ID NO:20, and a termination codon downstream of T7 promotor and ribosome binding sequence. Competent cells of *Escherichia coli* strain "BL21(DE3)pLysS", employed in Example 1-3(a), were transformed in usual manner with the recombinant DNA "pEscFv#125-2H.HT" to obtain a transformant. Thus-obtained transformant was named "EscFv#125-2H.HT".

### Example 2-2

### Production of peptide by transformant

The transformant "EscFv#125-2H.HT", obtained in Example 2-1, was cultivated correspondingly to Example 1-3(b) in a 100ml scale. Collection of cells from the culture, collection of the inclusion body fraction after disrupting the cells, and wash of the inclusion body fraction were carried out similarly as in Example 1-3(b) to obtain the washed inclusion body fraction. To the washed inclusion body fraction, 10% volume of 0.1M Tris-HCl buffer (pH7.0) containing 6M guanidine hydrochloride (hereinafter called "6M guanidine-HCl solution") was added and stirred at 4°C overnight to solubilize the inclusion bodies. The solubilization product was applied to a column of 5ml affinity chromatography gel "Ni-NTA agarose", commercialized by QIAGEN GmbH, Hilden, Germany, and through the column 6M guanidine-HCl solution and 25mM Tris-HCl buffer (pH7.0) containing 50mM imidazole and 6M urea were run in this order to remove non-adsorbed components. Then 25mM Tris-HCl buffer (pH7.0) containing 250mM imidazol and 6M urea was run through the column to elute and collect adsorbed components. The collected fraction was diluted with 50mM Tris-HCl buffer (pH7.0) containing 6M urea to give a protein concentration of less than 0.1mg/ml and then dialyzed at 4°C against 0.1M Tris-HCl buffer (pH7.0) containing 0.4M L-arginine-HCl and 2mM EDTA (hereinafter called "TAE buffer") to renature the proteinaceous components. After the dialysis was repeated thrice, dialysis was further conducted against TAE buffer containing 10mM oxidized glutathione at 4°C for six days. The dialyzed product was concentrated by ultrafiltration and then dialyzed against PBS. Analysis by conventional SDS-PAGE revealed that the dialyzed product contained a peptide of about 29kDa in a purity of about 95% or higher. The dialyzed product was lyophilized, resulting in a solid containing about lmg of the peptide.

The solid was dissolved in RPMI1640 medium supplemented with 10%(v/v) fetal calf serum to give desired various peptide concentrations for the test samples, which were then examined by the test in Example 1-1(a) for IL-18-neutralizing activity. The monoclonal antibody "#125-2HmAb" was also prepared similarly as in Example 1-1(b) and diluted to give desired various antibody concentrations with the same medium for the test samples, which were examined as above. After the test, IFN-γ amounts measured in the testing systems were calculated for percentages to that of control to estimate percent inhibition of the induction of IFN-γ by IL-18. The results are in FIG.5.

As shown in FIG.5, the peptide of this Example dose-dependently and effectively inhibited the IL-18 biological activity to induce IFN-γ production from KG-1 cells. The molecular weight of the peptide of this Example estimated by SDS-PAGE well coincided with the calculated molecular weight of the amino acid sequence of SEQ ID NO:10, about 29kDa. These results indicate that the peptide is a type of the peptide of this invention, having the amino acid sequence of SEQ ID NO:10, an artificially produced peptide which neutralizes IL-18 and contains a part or the whole of the amino acid sequences of SEQ ID NOs:1 and 2, of the variable regions in anti-IL-18 antibody. The results in FIG.5 also shows that the peptide of this Example exhibited IL-18-neutralizing activity with nearly equivalent efficiency to the monoclonal antibody "#125-2HmAb" in about twice mol concentration of the antibody. While the antibody belongs to IgG₁ to have two antigen-binding sites per molecule, the peptide of this Example is considered to have one. The results, therefore, indicate that the peptide of this Example neutralizes IL-18 with nearly equivalent efficiency to the parental antibody, and that the amino acid sequences of SEQ ID NOs:1 and 2 are partly or wholly useful in artificial producing of IL-18-neutralizing peptides. These results also indicate that the DNA obtained in this Example is a type of the DNA of this invention, coding for the present peptide, and the DNA facilitates the production of the peptide by the process using the DNA. In addition, when examined similarly as in Example 1-3(d) for binding to IL-18, the peptide of this invention specifically bound to IL-18.

### Example 3

### Peptide and DNA coding for the peptide

A type of the peptide of this invention in the form of a chimeric antibody is produced as follows. A DNA containing the nucleotide sequence coding for the constant region on human immunoglobulin light chain (K chain) is first isolated from human genomic library in accordance with the procedures by P. A. Hieter et al., in "Cell", Vol.22, pp.197-207 (1980). By conventional PCR using the isolated DNA as template, a DNA is prepared to substantially consist of the nucleotide sequence coding for the constant region, hereinafter called "human light chain constant region DNA". By PCR similarly as PCR A in Example 1-2, another DNA is prepared to have a sequence consisting of the nucleotides 1-384 of SEQ ID NO:27, hereinafter called "mouse light chain variable region DNA". Using the PCR-prepared DNAs as template, the method designated "overlap extension", described in Robert M. Horton, "Methods in Enzymology", Vol.217, pp.270-279 (1993), is conducted to prepare a DNA comprising the mouse light chain variable region DNA followed by the human light chain constant region DNA and restriction enzyme recognition sites positioned at the 5'- and 3'-termini. A DNA for an expression vector which contains, like as "pSV2-neo" (ATCC 37149), a replication origin in *Escherichia coli,* a promotor and/or enhancer functioning in a mammalian cell, restriction enzyme recognition sites in regulatable position thereby, selection sequences, etc., is then prepared. The expression vector and the above-prepared DNA comprising the human light chain constant region DNA and mouse light chain variable region DNA are subjected to restriction enzyme digestion followed by ligation using ligase to obtain a recombinant DNA containing a sequence coding for a chimeric antibody light chain.

A DNA containing the nucleotide sequence coding for the constant region on human immunoglobulin heavy chain (γ chain) is isolated from human genomic library in accordance with the procedures by N. Takahashi et al., in "Cell", Vol.29, pp.671-679 (1982). The isolated DNA comprises four independent exons as described in the paper. Using the isolated DNA as template, the above-mentioned "overlap extension" is conducted to prepare a DNA with the exons directly connected, hereinafter called "human heavy chain constant region DNA". By PCR similarly as PCR B in Example 1-2, another DNA is prepared to have a sequence consisting of the nucleotides 1-423 of SEQ ID NO:28, hereinafter called "mouse heavy chain variable region DNA". Using the PCR-prepared DNAs as template, the above-mentioned "overlap extension" is conducted to prepare a DNA comprising the mouse heavy chain variable region DNA followed by the human heavy chain constant region DNA and restriction enzyme recognition sites positioned at the 5'- and 3'-termini. A DNA for an expression vector which contains, like as "pSV2-gpt" (ATCC 37145), a replication origin in *Escherichia coli,* a promotor and/or enhancer functioning in a mammalian cell, restriction enzyme recognition sites in regulatable position thereby, selection sequences, etc., is then prepared. The expression vector and the above-prepared DNA comprising the human light chain constant region DNA and mouse light chain variable region DNA are subjected to restriction enzyme digestion followed by ligation using ligase to obtain a recombinant DNA containing a sequence coding for a chimeric antibody heavy chain.

The recombinant DNAs containing the sequences for the chimeric antibody heavy and light chains are next co-introduced by electroporation into mammalian established cell line such as CHO-Kl, ATCC CCL-61. The DNA-introduction product is screened on the basis of the selection sequences on the expression vectors, and the selected cells are independently cultivated. The culture supernatants are examined by the test in Example 1-1(a) for IL-18-neutralizing activity. Cells which produce the positive culture supernatants are subjected to limit dilution into a single cell to obtain a transformant which produces the peptide of this invention in the form of a chimeric antibody. The transformant is cultivated in larger scale, and the culture supernatant is subjected to conventional methods for antibody purification to obtain the peptide, in the form of a chimeric antibody. The peptide thus obtained effectively neutralizes IL-18 similarly as the anti-IL-18 monoclonal antibody "#125-2HmAb". The DNA according to this Example can be changed in sequences for the framework structures to code for similar amino acid sequences to the case of an human antibody obtainable from conventional databases by homology search with the peptide of this Example, and the changed DNA can be expressed to obtain another type of the peptide in the form of a humanized antibody comprising human framework structures. The humanized antibody thus obtainable can be predicted on three dimensional structure based on the amino acid sequence using conventional computational programs for protein structure analysis, and the predicted structure can be compared with the structure of the monoclonal antibody "#125-2HmAb" similarly predictable. Then the DNA for the humanized antibody can be further changed to express a three dimensional structure more closely resembled to the monoclonal antibody "#125-2HmAb", leading to obtainment of a humanized antibody which can exhibits substantially equivalent functions to the parental monoclonal antibody, "#125-2HmAb". The peptide of this Example and the peptides in the form of a humanized antibody form obtainable therefrom are useful in the treatment of the susceptive diseases.

### Example 4

### Liquid Agent

Peptides were prepared in accordance with the methods in Examples 1 and 2. Either of the peptide was dissolved to give a concentration of 1mg/ml in physiological saline containing as stabilizer 1%(w/v) powdered trehalose crystals "TREHAOSE®", commercialized by HAYASHIBARA Co., Ltd., Okayama, Japan, and sterilized in usual manner by membrane filtration to obtain a liquid agent.

The products are excellent in stability and useful in an injection, ophthalmic solution, collunarium, etc., to treat and prevent the susceptive diseases including autoimmune diseases.

### Example 5

### Dried injection

Peptides were prepared in accordance with the methods in Examples 1 and 2. One hundred milligrams of either of the peptide was dissolved in 100ml of physiological saline containing 1%(w/v) sucrose as stabilizer. The solution was sterilized in usual manner by membrane filtration and divided into aliquotes of 1ml per vial, which were lyophilized before sealing.

The products are excellent in stability and useful as a dried injection to treat and prevent the susceptive diseases including autoimmune diseases.

### Example 6

### Ointment

Carboxyvinylpolymers "HI-BIS-WAKO 104", commercialized by WAKO PURE CHEMICALS, Tokyo, Japan, and powdered trehalose crystals "TREHAOSE®", commercialized by HAYASHIBARA Co., Ltd., Okayama, Japan, were dissolved in sterilized distilled water to give respective concentrations of 1.4%(w/w) and 2.0%(w/w). The solution was mixed to homogeneity with either of peptides prepared in accordance with the methods in Examples 1 and 2 and adjusted to pH7.2 to obtain a paste containing lmg of the present peptide per 1g.

The products are excellent in spreadability and stability and useful as an ointment to treat and prevent the susceptive diseases including autoimmune diseases.

### Example 7

### Tablets

Powdered anhydrous α-maltose crystals "FINETOSE®", commercialized by HAYASHIBARA Co., Ltd., Okayama, Japan, was mixed to homogeneity with either of peptides prepared in accordance with the methods in Examples 1 and 2 and cell activating agent "LUMIN", [bis-4-(1-ethylquinoline)][γ-4'-(1-ethylquinoline)], and the resulting mixture was tabletted in usual manner to obtain tablets containing lmg of the present peptide and 1mg of "LUMIN" per tablet.

The products, with swallowability, stability, and cell activating property, are useful as tablets to treat and prevent the susceptive diseases including autoimmune diseases.

### Experiment

### Acute Toxicity Test

Each agent in accordance with Examples 4-7 was administered in usual manner to 8-week-old mice through percutaneous, peroral, or intraperitoneal route. In any route, LD₅₀ of the tested samples were about lmg/kg-body-weight or higher on the present peptide basis. These results support the safeness of the present peptide incorporated in pharmaceuticals directed to the uses for mammals including humans.

As explained above, this invention is based on artificially production of the peptides which effectively neutralize a biological activity of IL-18. The present peptide is efficacious in the alleviation of rejection reaction relating to grafting organs and the treatment and prevention of various diseases caused by excessive immunoreactions because the peptide suppresses and regulates immunoreactions of mammals including humans. The inhibitor, inhibition method, neutralizer, and neutralization method of this invention, which use the present peptide, are effectively used to treat various diseases directly or indirectly involving IL-18 biological activities and to suppress rejection reaction and excessive immunoreactions caused by grafting organs. The present peptide with such usefulness is easily produced in desired amounts by the process of this invention. Furthermore, the present peptide is useful for a reagent to screen for agonists and antagonists to IL-18.

This invention exhibits these remarkable effects and greatly contributes to the art.

While there has been described what is at present considered to be the preferred embodiments of this invention, it will be understood the various modifications may be made therein, and it is intended to cover in the appended claims all such modifications as fall within the true spirits and scope of the invention.

## Claims

1. An artificially produced peptide which neutralizes a biological activity of interleukin-18, comprising a part or the whole of the amino acid sequences of variable regions in anti-interleukin-18 antibody.

2. The peptide of claim 1, wherein the anti-interleukin-18 antibody is a monoclonal antibody.

3. The peptide of claim 1, wherein the anti-interleukin-18 antibody is against human or mouse interleukin-18 as antigen.

4. The peptide of claim 1, which suppresses inflammation induced by the biological activity of interleukin-18.

5. The peptide of claim 1, wherein the variable regions comprise the amino acid sequences of SEQ ID NOs:1 and 2.

6. The peptide of claim 1, which comprises a part or the whole of the amino acid sequences of complementarity determining regions in the variable regions.

7. The peptide of claim 1, which comprises a part or the whole of the amino acid sequences of SEQ ID NOs:3 to 8.

8. The peptide of claim 1, which has an amino acid sequence selected from the group consisting of SEQ ID NOs:9 and 10.

9. The peptide of claim 1, which is in the form of a humanized antibody.

10. A DNA which codes for the peptide of claim 1.

11. The DNA of claim 10, which comprises a part or the whole of a nucleotide sequence selected from the group consisting of SEQ ID NOs:11 and 12 and their complementary sequences.

12. The DNA of claim 10, which comprises a part or the whole of a nucleotide sequence selected from the group consisting of SEQ ID NOs:13 to 18 and their complementary sequences.

13. The DNA of claim 10, which has a nucleotide sequence selected from the group consisting of SEQ ID NOs:19 and 20 and their complementary sequences.

14. The DNA of claim 10, wherein at least one nucleotides are replaced by different ones, on the basis of genetic degeneracy, without changing the amino acid sequence encoded thereby.

15. The DNA of claim 10, which is inserted into an autonomously replicable vector.

16. The DNA of claims 10, which is introduced into a host selected from the group consisting of animal, plant, and microbial hosts.

17. A process of producing a peptide comprising the steps of allowing a DNA that codes for the peptide of claim 1 to express and collecting the expressed peptide.

18. The process of claim 17, wherein the peptide is collected by one or more techniques selected from the group consisting of salting out, dialysis, filtration, concentration, separatory sedimentation, ion-exchange chromatography, gel filtration chromatography, absorption chromatography, isoelectric-focusing chromatography, hydrophobic chromatography, reversed phase chromatography, affinity chromatography, gel electrophoresis, and isoelectric-focusing electrophoresis.

19. An agent for susceptive diseases, which comprises the peptide of claim 1 as an effective ingredient.

20. The agent of claim 19, which contains as a stabilizer one or more members selected from the group consisting of albumin, saccharides, and buffers.

21. The agent of claim 19, which is as an agent for autoimmune diseases.

22. The agent of claim 19, which is an immunosuppressant.

23. The agent of claim 19, which is an anti-inflammation agent.

24. An interleukin-18 neutralizer, which comprises the peptide of claim 1 as an effective ingredient.

25. A method of neutralizing interleukin-18, which comprises a step of allowing the peptide of claim 1 to act on interleukin-18.

26. An interleukin-18 inhibitor, which comprises the peptide of claim 1 as an effective ingredient.

27. A method of inhibiting interleukin-18, which comprises a step of allowing the peptide of claim 1 to act on interleukin-18.
